(19) Europäisches Patentamt
European Patent Office
Office européen des brevets

(11) **EP 3 081 157 A1**

(12) **EUROPEAN PATENT APPLICATION**

(43) Date of publication:
**19.10.2016 Bulletin 2016/42**

(51) Int Cl.:
**A61B 5/024** (2006.01)    A61B 5/046 (2006.01)
A61B 5/11 (2006.01)    A61B 5/16 (2006.01)

(21) Application number: **16165590.7**

(22) Date of filing: **15.04.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.04.2015   JP 2015084708**
**17.04.2015   JP 2015084709**

(71) Applicant: **Seiko Epson Corporation**
**Tokyo 160-8801 (JP)**

(72) Inventors:
• **Narusawa, Atsushi**
**Nagano, 392-8502 (JP)**
• **Watanabe, Shinichiro**
**Nagano, 392-8502 (JP)**

(74) Representative: **Hoffmann Eitle**
**Patent- und Rechtsanwälte PartmbB**
**Arabellastraße 30**
**81925 München (DE)**

(54) **BIOLOGICAL INFORMATION PROCESSING SYSTEM, BIOLOGICAL INFORMATION PROCESSING DEVICE, TERMINAL DEVICE, METHOD FOR GENERATING ANALYSIS RESULT INFORMATION, AND BIOLOGICAL INFORMATION PROCESSING METHOD**

(57)    A biological information processing system includes a processor including hardware. The processor performs: acquisition processing in which pulse wave information is acquired; processing in which analysis processing on the pulse wave information is performed to generate analysis result information; and output processing in which the analysis result information that is generated is outputted. The processor generates the analysis result information for identifiably displaying a pulse wave abnormality period detected on the basis of the analysis processing and for displaying at least one of action information, psychological state information, external environment information and location information of a user.

FIG. 1

**Description**

<u>CROSS-REFERENCE</u>

This application is based upon Japanese Patent Application No. 2015-084708 filed on April 17, 2015 and Japanese Patent Application No. 2015-084709 filed on April 17, 2015, the entire contents of which are incorporated herein by reference.

<u>BACKGROUND</u>

1. Technical Field

The present invention relates to a biological information processing system, a biological information processing device, a terminal device, a method for generating analysis result information, and a biological information processing method and the like.

2. Related Art

Arrhythmias are widely known as cardiovascular problems. For example, atrial fibrillation, which is an example of arrhythmias, needs to be treated in early stages because of the risk of blood clotting in the heart and consequent cerebral infarction. Traditionally, in a test to diagnose atrial fibrillation, it is common to have the patient wear a Holter electrocardiograph for 24 to 48 hours (usually 24 hours) and check the occurrence of atrial fibrillation from the electrocardiogram taken during that period.

However, since atrial fibrillation in its initial stage has a low frequency of occurrence, atrial fibrillation that occurs only occasionally can be overlooked in a Holter electrocardiographic test for a duration of 48 hours or less. Also, a patient with asymptomatic atrial fibrillation does not experience any symptoms and therefore, in many cases, does not visit a hospital and finds out that he/she has atrial fibrillation only after the condition becomes severe (for example, after cerebral infarction occurs). The reason for not being able to use a Holter electrocardiograph for a long period of time is that a plurality of electrodes needs to be attached to the chest of a human body and this raises the problem of skin rash caused by the electrodes themselves and tapes used to hold the electrodes. While electrocardiographs that can record data for 40 days have been developed recently, the problem of skin rash has not been solved and therefore it is not realistic to use even these electrocardiographs continuously for a long period of time.

In contrast, JP-A-2013-55982 discloses a technique in which a parameter equivalent to RR interval is found accurately from pulse wave information, thus finding atrial fibrillation on the basis of the pulse wave information. The pulse wave information can be acquired by a pulse wave sensor such as a photoelectric sensor, for example. A device having the pulse wave sensor can be implemented, for example, as a wristwatch-type wearable device. Therefore, it is possible to wear the device continuously for a long period of time, while restraining the possibility of skin rash or the like.

By using pulse wave information as in JP-A-2013-55982 or the like, it is possible to wear the device over a long period of time (for example, about three to ten days) and therefore it is possible to properly detect atrial fibrillation or the like in its initial stage.

<u>SUMMARY</u>

An aspect of the invention relates to a biological information processing system including a processor including hardware. The processor performs: acquisition processing in which pulse wave information is acquired; processing in which analysis processing on the pulse wave information is performed to generate analysis result information; and output processing in which the analysis result information that is generated is outputted. The processor generates the analysis result information for identifiably displaying a pulse wave abnormality period detected on the basis of the analysis processing and for displaying at least one of action information, psychological state information, external environment information and location information of a user.

Another aspect of the invention relates to a biological information processing system including a processor including hardware. The processor performs: acquisition processing in which pulse wave information is acquired; processing in which analysis processing on the pulse wave information is performed to generate analysis result information; and display processing on the analysis result information. The processor, in the display processing, performs display processing on a first screen where the analysis result information is displayed, and performs the display processing on a second screen where original information used to generate the analysis result information is displayed, when a user carries out a predetermined instruction operation.

Still another aspect of the invention relates to a biological information processing device including a processor including hardware. The processor performs: acquisition processing in which pulse wave information is acquired; processing in which analysis processing on the pulse wave information is performed to generate analysis result information; and output processing in which the analysis result information that is generated is outputted. The processor generates the analysis result information for identifiably displaying a pulse wave abnormality period detected on the basis of the analysis processing and for displaying at least one of action information, psychological state information, external environment information and location information of a user.

Yet another aspect of the invention relates to a terminal device including a processor including hardware, and a display. The processor performs: reception processing in which analysis result information about pulse wave information is received; and display processing at the display on the analysis result information that is received. The processor performs display processing on a first screen where the analysis result information is displayed, and performs the display processing on a second screen where original information used to generate the analysis result information is displayed, when a user carries out a predetermined instruction operation.

Still yet another aspect of the invention relates to a method for generating analysis result information including: performing processing in which pulse wave information is acquired; and performing analysis processing on the pulse wave information and thus generating analysis result information for identifiably displaying a pulse wave abnormality period detected on the basis of the analysis processing and for displaying at least one of action information, psychological state information, external environment information and location information of a user.

Further another aspect of the invention relates to a biological information processing method including: acquiring pulse wave information; performing processing in which analysis processing on the pulse wave information is performed to generate analysis result information; performing display processing on a first screen where the analysis result information that is generated is displayed; and performing the display processing on a second screen where original information used to generate the analysis result information is displayed, when a user carries out a predetermined instruction operation.

## BRIEF DESCRIPTION OF THE DRAWINGS

The invention will be described with reference to the accompanying drawings, wherein like numbers reference like elements.

FIG. 1 shows an example of the configuration of a biological information processing system according to an embodiment.
FIG. 2 shows an example of information displayed by analysis result information.
FIGS. 3A to 3C show an example of action information displayed by analysis result information.
FIG. 4 shows another example of information displayed by analysis result information.
FIGS. 5A to 5D show another example of information displayed by analysis result information.
FIG. 6 is an explanatory view showing processing of dinging temperature difference information.
FIG. 7 shows another example of information displayed by analysis result information.
FIGS. 8A and 8B show another example of information displayed by analysis result information.
FIG. 9 shows another example of information displayed by analysis result information.
FIGS. 10A and 10B show another example of information displayed by analysis result information.
FIG. 11 shows an example of information displayed by second analysis result information.
FIG. 12 shows an example of information displayed by second analysis result information.
FIG. 13 shows another example of information displayed by second analysis result information.
FIG. 14 shows an example of analysis report displayed by analysis result information.
FIG. 15 shows an example of analysis report displayed by analysis result information.
FIG. 16 shows an example of the detailed configuration of a biological information processing system according to an embodiment.
FIG. 17 shows an example of the configuration of an analysis processor.
FIGS. 18A and 18B are graphs showing result of frequency analysis processing based on electrocardiogram RR interval.
FIG. 19 is a flowchart explaining a flow of analysis processing according to an embodiment.
FIG. 20 is an explanatory view showing a technique for setting determination sections overlapping each other.
FIGS. 21A and 21B show an example of the configuration of a pulse wave measuring device.
FIG. 22 shows an example of the configuration of a pulse wave measuring device.
FIGS. 23A to 23C show an example of the configuration of a pulse wave measuring device and a biological information processing system.
FIG. 24 shows an example of displaying second analysis result information, using a new display window.

DESCRIPTION OF EXEMPLARY EMBODIMENTS

An embodiment relates to a biological information processing system including a processor including hardware. The processor performs: acquisition processing in which pulse wave information is acquired; processing in which analysis processing on the pulse wave information is performed to generate analysis result information; and output processing in which the analysis result information that is generated is outputted. The processor generates the analysis result information for identifiably displaying a pulse wave abnormality period detected on the basis of the analysis processing and for displaying at least one of action information, psychological state information, external environment information and location information of a user.

In the embodiment, the processor generates the analysis result information for identifiably displaying a pulse wave abnormality period and for displaying at least one of various kinds of information. Thus, since the action information or the like can be displayed in association with the pulse wave abnormality period, it is possible to facilitate, for example, determination (diagnosis) by the viewer on an occurrence factor of an arrhythmia corresponding to the pulse wave abnormality period, or the like.

Also, in the embodiment, the processor may generate the analysis result information for displaying the action information which is information indicating whether the user is in a sleeping state or in an awake state.

Thus, it is possible to display the action information indicating whether the user is in the sleeping state or in the awake state, or the like.

Also, in the embodiment, the processor may generate the analysis result information for displaying the action information as information for determination on whether a sympathetic nerve-dependent arrhythmia or a parasympathetic nerve-dependent arrhythmia occurs during the pulse wave abnormality period.

Thus, by displaying the action information indicating whether the user is in the sleeping state or in the awake state, it is possible to facilitate determination on an occurrence factor of an arrhythmia, or the like.

Also, in the embodiment, the processor may generate the analysis result information for displaying the psychological state information which is stress information of the user.

Thus, it is possible to display the psychological state information which is stress information, or the like.

Also, in the embodiment, the processor may generate the analysis result information for displaying the psychological state information as information for determination on whether a stress-induced arrhythmia occurs during the pulse wave abnormality period or not.

Thus, by displaying the psychological state information which is stress information, it is possible to facilitate determination on an occurrence factor of an arrhythmia, or the like.

Also, in the embodiment the processor may generate the analysis result information for displaying the external environment information which is at least one of temperature information, barometric pressure information, external light information, and ambient sound information.

Thus, it is possible to display at least one of various kinds of external environment information, or the like.

Also, in the embodiment, the processor may generate the analysis result information for displaying the external environment information which is one of the temperature information for determination on a temperature-induced arrhythmia, the barometric pressure information for determination on a barometric pressure-induced arrhythmia, the external light information for determination on an external light-induced arrhythmia, and the ambient sound information for determination on an ambient sound-induced arrhythmia, as information for determination on what external environment induces an arrhythmia occurring during the pulse wave abnormality period.

Thus, by displaying the external environment information, it is possible to facilitate determination on an occurrence factor of an arrhythmia, or the like.

Also, in the embodiment, the temperature information may be temperature difference information between body surface temperature of the user and external temperature.

Thus, by using the temperature difference information as the temperature information, it is possible to display information for allowing accurate execution of determination on a temperature-induced arrhythmia.

Also, in the embodiment, the processor may generate information for displaying at least one of a pulse waveform, a pulse interval waveform and pulse interval distribution information during a measurement period, as the analysis result information.

Thus, it is possible to display information about the pulse wave of the user as well as the result of determination on the pulse wave abnormality period, or the like.

Also, in the embodiment, the processor may generate information for displaying a reliability index of measurement of the pulse wave information during the measurement period, as the analysis result information.

Thus, it is possible to display the reliability index along with the display of the pulse wave abnormality period.

Also, in the embodiment, the processor may generate information for displaying a display object indicating the pulse wave abnormality period, as the analysis result information.

Thus, it is possible to identifiably display the pulse wave abnormality period, by using the display object.

Also, in the embodiment, the processor may generate information for displaying the pulse wave abnormality period and a period that is not the pulse wave abnormality period, in different image display forms from each other, as the analysis result information.

Thus, it is possible to identifiably display the pulse wave abnormality period, by changing the image display form.

Also, in the embodiment, the processor may generate second analysis result information for displaying at least one of a pulse waveform and a pulse interval waveform during a predetermined section in the pulse wave abnormality period.

Thus, it is possible to display more detailed information during the pulse wave abnormality period, using the second analysis result information, or the like.

Another embodiment of the invention relates to a biological information processing system including a processor including hardware. The processor performs: acquisition processing in which pulse wave information is acquired; processing in which analysis processing on the pulse wave information is performed to generate analysis result information; and display processing on the analysis result information. The processor, in the display processing, performs display processing on a first screen where the analysis result information is displayed, and performs the display processing on a second screen where original information used to generate the analysis result information is displayed, when a user carries out a predetermined instruction operation.

In this another embodiment of the invention, display processing is performed on a first screen where the analysis result information is displayed, and when the user carries out a predetermined instruction operation, the display processing is performed on a second screen where original information used to generate the analysis result information is displayed. Thus, it is possible to switch between the analysis result information about the pulse wave information and the original information of the analysis result information, as the information to be displayed.

Also, in this another embodiment of the invention, the processor, in the display processing, may perform the display processing in which a pulse wave abnormality period for determination on an abnormality in a cardiovascular system is identifiably displayed as the analysis result information on the first screen, and may perform the display processing in which pulse interval information is displayed as the original information on the second screen.

Thus, it is possible for the viewer to view, on the second screen, the pulse interval information corresponding to the pulse wave abnormality period displayed on the first screen, or the like.

Also, in this another embodiment of the invention, the processor, in the display processing, may perform the display processing in which the pulse interval information corresponding to the pulse wave abnormality period is displayed on the second screen, when the predetermined instruction operation by the user is carried out on a display object indicating the pulse wave abnormality period displayed on the first screen.

Thus, it is possible to display the pulse interval information corresponding to the pulse wave abnormality period which the viewer wants to view, or the like.

Also, in this another embodiment of the invention, the processor, in the display processing, may perform the display processing in which the pulse interval information corresponding to a predetermined section in the pulse wave abnormality period is displayed on the second screen, when the predetermined instruction operation by the user is carried out on the display object indicating the pulse wave abnormality period displayed on the first screen.

Thus, it is possible to display the pulse interval information corresponding to the section which the user wants to view, of the pulse wave abnormality period, or the like.

Also, in this another embodiment of the invention, the processor may decide the predetermined section on the basis of reliability of pulse wave measurement.

Thus, it is possible to display the pulse interval information corresponding to a period with high reliability, for example, without displaying the pulse interval information corresponding to a period with low reliability in pulse wave measurement, or the like.

Also, in this another embodiment of the invention, the processor may generate information for displaying the pulse wave abnormality period and a pulse wave normality period which is a period that is not the pulse wave abnormality period, in different implementation forms from each other, as the analysis result information.

Thus, it is possible for the viewer to easily discriminate between the pulse wave abnormality period and the pulse wave normality period, or the like.

Also, in this another embodiment of the invention, the abnormality in the cardiovascular system may be an arrhythmia.

Thus, it is possible for the viewer to confirm the pulse wave abnormality period during which an arrhythmia occurs in the subject and the pulse interval information corresponding to the pulse wave abnormality period, or the like.

Also, in this another embodiment of the invention, the processor, in the display processing, may perform the display processing in which a reliability index of pulse wave measurement found on the basis of body motion information of the user is displayed on the first screen as the analysis result information, and may perform the display processing in which the body motion information is displayed on the second screen as the original information.

Thus, when a plurality of pulse wave abnormality periods is displayed by the analysis result information, a period with higher reliability, that is, a period which is suspected of having the occurrence of an abnormality in the user himself/herself instead of noise, can be preferentially used for diagnosis, and the burden of diagnosis can be reduced.

Also, in this another embodiment of the invention, the processor, in the display processing, may perform the display processing in which pulse rate information is displayed on the first screen as the analysis result information, and may perform the display processing in which pulse waveform information is displayed on the second screen as the original information.

Thus, it is possible for the viewer to view, on the second screen, the pulse waveform information corresponding to the pulse rate information displayed on the first screen, or the like.

Also, in this another embodiment of the invention, the predetermined instruction operation may be at least one of a click operation, a touch operation, a tap operation, a pinch-in operation, a pinch-out operation, a scroll operation, and a swipe operation on the first screen.

Thus, it is possible for the viewer to easily designate the pulse wave abnormality period for which the viewer wants to view the pulse interval information, or the like.

Also, in this another embodiment of the invention, the processor, in the display processing, may perform the display processing in which at least one of a total pulse rate per day, an average pulse rate per day, a rate of a total duration of occurrence of atrial fibrillation to a duration of test, a total duration of occurrence of atrial fibrillation per day, a ratio of the total duration of occurrence of atrial fibrillation per day to the duration of test, and a rate of occurrence of atrial fibrillation in a designated time bracket, is displayed.

Thus, it is possible to display information useful for the doctor to determine the frequency of occurrence of atrial fibrillation or whether the occurrence of atrial fibrillation concentrates on a specific day or not, or the like.

Also, in this another embodiment of the invention, the processor, in the display processing, may perform the display processing in which the analysis result information corresponding to an entire duration of test or the analysis result information corresponding to a predetermined period is displayed on the first screen.

Thus, it is possible to display, on the first screen, the analysis result information corresponding to the period which the viewer wants to view, or the like.

Also, in this another embodiment of the invention, the processor, in the display processing, may display a mark at a position on the first screen corresponding to a timing when an operation carried out when the user becomes aware of a pulse wave abnormality is detected.

Thus, it is possible for the doctor to determine whether it is an arrhythmia or not, after grasping the timing when the subj ect himself/herself feels a pulse wave abnormality, or the like.

Still another embodiment of the invention relates to a biological information processing device including a processor including hardware. The processor performs: acquisition processing in which pulse wave information is acquired; processing in which analysis processing on the pulse wave information is performed to generate analysis result information; and output processing in which the analysis result information that is generated is outputted. The processor generates the analysis result information for identifiably displaying a pulse wave abnormality period detected on the basis of the analysis processing and for displaying at least one of action information, psychological state information, external environment information and location information of a user.

Still another embodiment of the invention relates to a terminal device including a processor including hardware, and a display. The processor performs: reception processing in which analysis result information about pulse wave information is received; and display processing at the display on the analysis result information that is received. The processor performs display processing on a first screen where the analysis result information is displayed, and performs the display processing on a second screen where original information used to generate the analysis result information is displayed, when a user carries out a predetermined instruction operation.

Still another embodiment of the invention relates to a method for generating analysis result information, the method including: performing processing in which pulse wave information is acquired; and performing analysis processing on the pulse wave information and thus generating analysis result information for identifiably displaying a pulse wave abnormality period detected on the basis of the analysis processing and for displaying at least one of action information, psychological state information, external environment information and location information of a user.

Still another embodiment of the invention relates to a biological information processing method including: acquiring pulse wave information; performing processing in which analysis processing on the pulse wave information is performed to generate analysis result information; performing display processing on a first screen where the analysis result information that is generated is displayed; and performing the display processing on a second screen where original information used to generate the analysis result information is displayed, when a user carries out a predetermined instruction operation.

Hereinafter, an embodiment will be described. The embodiment below should not unduly limit the contents of the invention described in the appended claims. Not all of the configurations described in the embodiment are necessarily essential components of the invention.

1. Technique in This Embodiment

First, a technique in this embodiment will be described. As disclosed in JP-A-2013-55982, a technique in which a pulse wave signal (photoelectric pulse wave signal, pulse wave information) acquired by a photoelectric transducer (photoelectric sensor) worn by a test subject is analyzed so as to conduct a test to diagnose an arrhythmia such as atrial fibrillation is known. Here, the test to diagnose an arrhythmia refers to detecting a part (period, section) in which an arrhythmia is suspected, in order for a doctor to make a diagnosis.

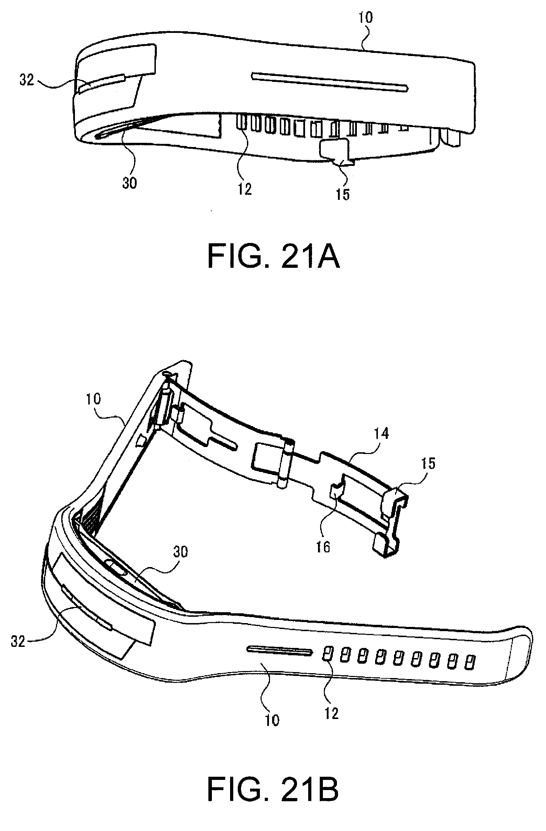

This test using a photoelectric pulse wave, compared with a test using a electrocardiograph, has an advantage that the device used is simpler and easier for the subject to wear, or that it suffices to press a device in the shape of a wristwatch or the like as shown in FIGS. 21A to 22 or the like in contact with the epidermis, causing less wearing load. This enables a longer-time test with an advantage that there is a high probability of catching a symptom onset phase that occurs irregularly. The site at which the pulse wave measuring device is fixed is not limited to the wrist and may be other sites such as the finger, neck, or ankle.

For example, atrial fibrillation is known as an arrhythmia. Atrial fibrillation is an abnormality in which a cardiac ventricle has convulsions (partial excitation and contraction), and the contraction of the cardiac ventricle occurs at irregular intervals. This causes the blood flow to stagnate. If atrial fibrillation continues for a long period of time, a thrombus can be formed easily. This can cause serious symptoms such as cerebral infarction and myocardial infarction.

Atrial fibrillation has a low frequency of occurrence in its initial stage. For example, it is possible that symptoms of atrial fibrillation appear only once in three days and for a few hours. In such a case, it is possible that the period of occurrence and the period when the Holter electrocardiograph is worn by the subject do not overlap with each other, resulting in an inability to detect atrial fibrillation. In contrast, a pulse wave measuring device (biological information processing device) for measuring pulse wave information can be easily worn for a longer period of approximately for three to ten days and therefore can detect atrial fibrillation even in the state where the frequency of occurrence is low.

Also, in the case of atrial fibrillation, asymptomatic atrial fibrillation of which the patient is unaware may be confirmed. In this case, it is unlikely that the patient will proactively have a test for atrial fibrillation. In some cases, there is a risk that symptoms may advance while the patient remains unaware of them, and may cause serious symptoms such as cerebral infarction. Even in this case, the use of pulse wave information is advantageous in that the patient can have a screening test with ease because the device can be easily worn for a long period.

The technique for detecting an arrhythmia (atrial fibrillation from pulse wave information will be described later. What is directly found by this technique is whether it is determined as a pulse wave abnormality period or not (whether atrial fibrillation is suspected or not) at each processing timing. However, for the viewer viewing the result of analysis of atrial fibrillation, it is desirable that not only such simple information but also more diverse information is presented, in order to verify the circumstances of occurrence and causes of occurrence from different perspectives.

For example, atrial fibrillation can be classified into sympathetic nerve-dependent atrial fibrillation (sympathetic nerve-dependent arrhythmia) and vagus nerve-dependent atrial fibrillation (parasympathetic nerve-dependent arrhythmia. The former tends to occur during the daytime or during activity, whereas the latter tends to occur during the night or during sleep. That is, if action information of the user is displayed in association with the result of determination on whether it is a pulse wave abnormality period or not, when the doctor diagnoses that there is an occurrence of atrial fibrillation during the pulse wave abnormality period, the displayed information can be used as a clue to specifying (classifying) the main factor in the atrial fibrillation. Since effective drugs for sympathetic nerve-dependent atrial fibrillation and parasympathetic nerve-dependent atrial fibrillation are different, if proper classification can be made, it is useful for making a decision on treatment approaches.

Also, since various other factors in an arrhythmia are conceivable, it is useful to present information that specifies these factors. Even a test using the traditional Holter electrocardiograph employs the method of making the patient write an action memo in a certain way. The action memo is a record of what action the user takes in which time bracket. The action memo may be prepared by the user writing on a sheet of paper or by the user operating an operation device provided the Holter electrocardiograph or the like. Preparing an action memo on a smartphone or the like owned by the user can become popular in the future. In any case, the traditional method assumes that the user actively prepares an action memo. This puts a heavy burden on the user and there are cases where no action memo is prepared in practice.

In view of such a burden on the user, the present applicant proposes a technique of acquiring information from various sensors or the like and preparing and displaying information for specifying factors in an arrhythmia on the basis of the acquired information. Thus, information equivalent to the action memo in the traditional method can be automatically prepared, and the prepared information can be presented to the viewer (doctor) in an intelligible form. Meanwhile, in the embodiment, making the user prepare an action memo as in the traditional method is not precluded. In such a case, information acquired from sensors or the like can be used as information that complements the action memo.

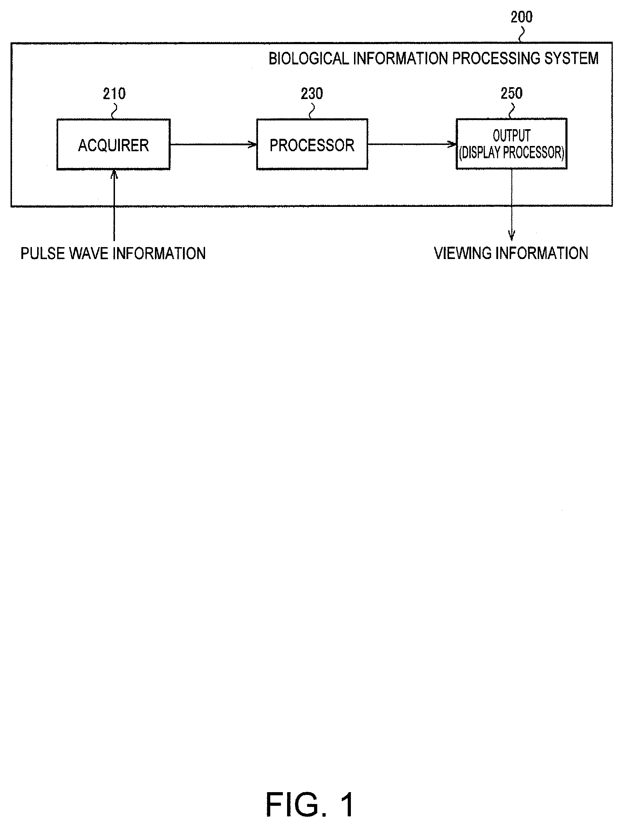

Specifically, a biological information processing system 200 according to the embodiment includes an acquirer 210 which acquired pulse wave information, a processor 230 which performs analysis processing of the pulse wave information and thus generates analysis result information, and an output 250 which outputs the generated analysis result information, as shown in FIG. 1. The processor 230 generates analysis result information for identifiably displaying a pulse wave abnormality period detected on the basis of the analysis processing and for displaying at least one of action information, psychological state information, external environment information and location information of the user.

Here, the pulse wave information is information indicating a pulse wave of a user (test subject, subject), and specifically, information based on an output from a pulse wave sensor (photoelectric sensor) worn by the user. The pulse wave abnormality period is a period in which it is detected that the pulse wave information is different from its normal state (in an abnormal state). An example of the pulse wave abnormality period is a period in which it is detected that the variation in the pulse interval is great, as described later. Details of the method for detecting the pulse wave abnormality period will be described later.

The processor 230 may generate analysis result information for identifiably displaying a pulse wave abnormality period for determining an abnormal state of the cardiovascular system during the measurement period. That is, the pulse wave abnormality period may be information indicating whether it is a period in which an abnormality in the cardiovascular system is suspected or not. Also, the abnormal state of the cardiovascular system may be an arrhythmia.

The pulse wave sensor such as a photoelectric sensor can detect, for example, a fluctuation in the blood flow rate in a blood vessel. That is, an abnormality in the cardiovascular system, mainly in the blood vessel system, can be detected on the basis of the analysis processing on the pulse wave information. With the analysis result information according to the embodiment, the abnormal state of the cardiovascular system can be intelligibly presented to the viewer. Moreover, since the use of information such as the pulse rate or the pulse interval enables determination on whether the pulse of the user is in a normal state or not, it is possible to focus on an arrhythmia as the abnormal state of the cardiovascular system. In the description below, it is assumed that the pulse wave abnormality period is for determining whether there is an arrhythmia or not. Also, the arrhythmia in the embodiment, in a narrow sense, is atrial fibrillation, as described later.

The action information of the user is information indicating the action of the user. For example, the action information is information specifying the type of activity or the amount of activity by the user. As the type of activity, a sleeping state, an awake state and the like may be employed. Also, mode detailed classification types may be set, such as further classifying the awake state into a resting state and an exercising state, or further classifying the exercising state into walking, running, and the like. As the amount of activity information, calories burned or oxygen intake may be used, or the unit of METs expressing the intensity of physical activity described in the Ministry of Health, Labour and Welfare's guide may be used. METs is information indicating the intensity of physical activity in the form of how many times the intensity of physical activity at rest.

The psychological state information of the user is information indicating the mental activity of the user. For example, the psychological state information is an index value indicating the degree of mental stress which the user is experiencing. The external environment information of the user is information indicating the state of the external (surrounding) environment of the user and includes various kinds of information such as temperature, barometric pressure, external light and sound, as descried later. The location information of the user is information indicating the place where the user is present (or was present) . The location information may be expressed, for example, by an absolute value based on latitude and longitude or the like. However, this is not limiting and abstracted information may also be used. For example, the location information may be information indicating an attribute of the place where the user is present, such as "home", "workplace", "station", and "amusement facilities".

Thus, various kinds of information useful for specifying factors in an arrhythmia can be displayed along with the result of detection of a pulse wave abnormality period. Therefore, when referring to detailed information or the like of a pulse wave abnormality period and diagnosing the occurrence of an arrhythmia during the pulse wave abnormality period, the doctor can easily presume a factor in the arrhythmia, or the like.

Specifically, the processor 230 may generate analysis result information for displaying action information which is information indicating whether the user is in a sleeping state or in an awake state. The action information in this case, that is, the information indicating whether the user is in a sleeping state or in an awake state, is information for determination on whether a sympathetic nerve-dependent arrhythmia or a parasympathetic nerve-dependent arrhythmia occurs during the pulse wave abnormality period or not.

The processor 230 may also generate analysis result information for displaying psychological state information which is stress information of the user. The psychological state information in this case, that is, the stress information of the user, is information for determination on whether a stress-induced arrhythmia occurs during the pulse wave abnormality period or not.

It is known that the stress experienced by the user is closely related with the user's arrhythmia. Therefore, if an arrhythmia can be determined as being caused by stress, solving the stress factor can improve the arrhythmia. The psychological state information in this example is, in a narrow sense, information indicating mental stress on the user. As an example, information indicating the degree (magnitude) of mental stress may be used.

In the case of generating analysis result information for displaying action information, the processor 230 may find the action information on the basis of pulse wave information. Also, in the case of generating analysis result information for displaying psychological state information, the processor 230 may generate the psychological state information on the basis of pulse wave information. Thus, action information and psychological state information can be found from pulse wave information. The method for finding these kinds of information from pulse wave information will be described later.

The processor 230 may also generate analysis result information for displaying external environment information which is at least one of temperature information, barometric pressure information, external light information, and ambient sound information. The temperature information in this example may be information for determination on whether there is a temperature-induced arrhythmia. The barometric pressure information may be information for determination on whether there is a barometric pressure-induced arrhythmia. The external light information may be information for determination on whether there is an external light-induced arrhythmia. The ambient sound information may be information for determination on whether there is an ambient sound-induced arrhythmia. The external environment information in this example, that is, at least one of the temperature information, the barometric pressure information, the external light information and the ambient sound information, is information for determination on what external environment induces an arrhythmia during the pulse wave abnormality period.

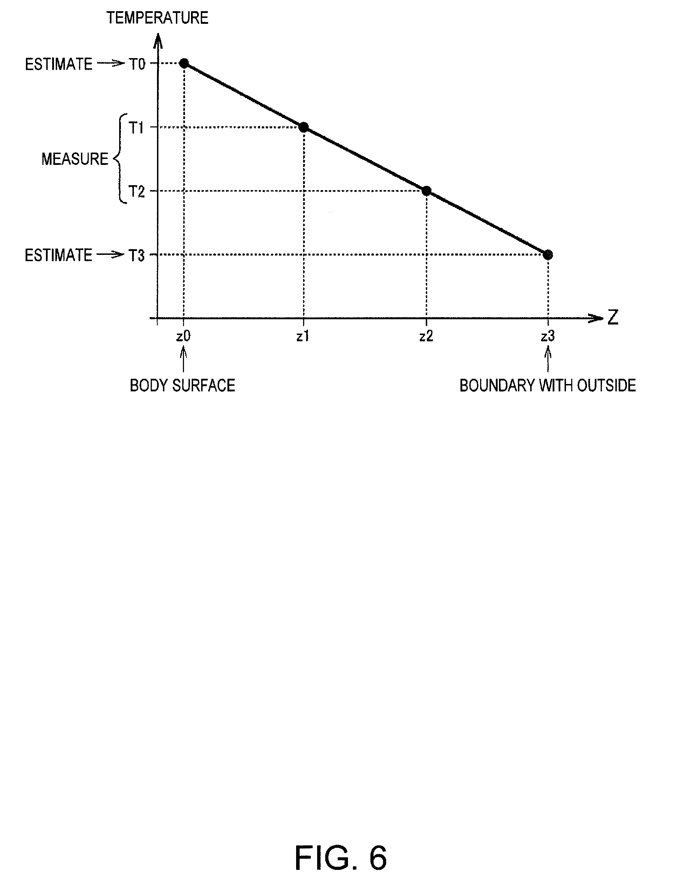

The temperature information is acquired, for example, by a temperature sensor included in the pulse wave measuring device, and to be more precise, it indicates the temperature inside the device where the temperature sensor is provided. In the embodiment, the temperature inside this device may be employed as the temperature information. However, the temperature information is not limited to this and may be temperature difference information between the body surface temperature of the user and the ambient temperature. The body surface temperature of the user refers to the temperature at a position corresponding to the skin, more specifically, the skin surface, of the body temperature of the user. The ambient temperature refers to the temperature in the external (surrounding) atmosphere around the user. As described later with reference to FIG. 6, by using a plurality of temperatures inside the device, it is possible to estimate the body surface temperature and the ambient temperature from the temperatures inside the device. That is, the body surface temperature and the ambient temperature in this example are not limited to the body surface temperature and the ambient temperature which are directly measured, and may be temperatures determined as corresponding to the body surface temperature and the ambient temperature.

The barometric pressure information is information indicating the barometric pressure acting on the user or the barometric pressure in the environment where the user is present. For example, the pulse wave measuring device may include a barometric pressure sensor (pressure sensor) and find the barometric pressure information on the basis of an output from the sensor. It is known that the user's health condition may get worse when barometric pressure drops because of bad weather such as a typhoon or because the user moves to a location with a high elevation above sea level. That is, a change (in a narrow sense, a drop) in barometric pressure puts a burden on the user and can be a factor in an arrhythmia. Therefore, by examining the correlation between the pulse wave abnormality period and the barometric pressure information, it is possible to determine whether the arrhythmia is induced by barometric pressure or not.

The external light information is information indicating the state of light in the surroundings of the user. Irradiation with excessively intense light, flashing light, or light in a specific color can cause the user to feel unwell such as having a headache or dizziness. Therefore, light can be a factor in an arrhythmia as well. The external light information in this example may be, ideally, information of light cast on the user, but in practice, it may be information of light cast on the pulse wave measuring device worn by the user. Specifically, the pulse wave measuring device may have an illuminance sensor and may acquire the external light information on the basis of an output from the illuminance sensor. Alternatively, the external light information may be acquired on the basis of an output from an image pickup element (photoelectric sensor) of a camera that is broadly used.

The ambient sound information is information about sounds in the surroundings of the user. It is known that noise can be harmful to health. Also, even if the loudness of a sound (for example, the numerical value of dB) is not high, a sound in a specific frequency band can cause a person to feel discomfort. Therefore, a sound can be a factor in an arrhythmia. The ambient sound information in this example may be, ideally, information of a sound wave entering the user's ears, but in practice, it may be information about a sound wave detected by the pulse wave measuring device worn by the user. Specifically, the pulse wave measuring device may have sound pickup sensor such as a microphone and acquire the ambient sound information on the basis of an output from the microphone or the like.

In the above description, it is assumed that the pulse wave measuring device is equipped with sensors for acquiring various kinds of information. However, this example is not limiting and the biological information processing system 200 may acquire information acquired by another device. For example, a mobile terminal device such as a smartphone used by the user may be equipped with the various sensors described above and acquire corresponding information.

The processor 230 may also generate information for displaying at least one of a pulse waveform, a pulse interval waveform and pulse interval distribution information during the measurement period, as the analysis result information.

Here, the pulse waveform is a waveform of pulse wave information, for example, a waveform indicating changes in time series in the pulse wave information. Specifically, the pulse waveform may be a waveform indicating an AC component of a pulse wave sensor output. Meanwhile, the pulse interval waveform is a waveform indicating changes in time series in the pulse interval. The pulse interval is a period of time indicating the interval between one pulse to another (for example, in msec). For example, in relation to pulse rate (in rpm), which is broadly used, the pulse interval is in the relation of pulse interval = (1000x60)/ pulse rate.

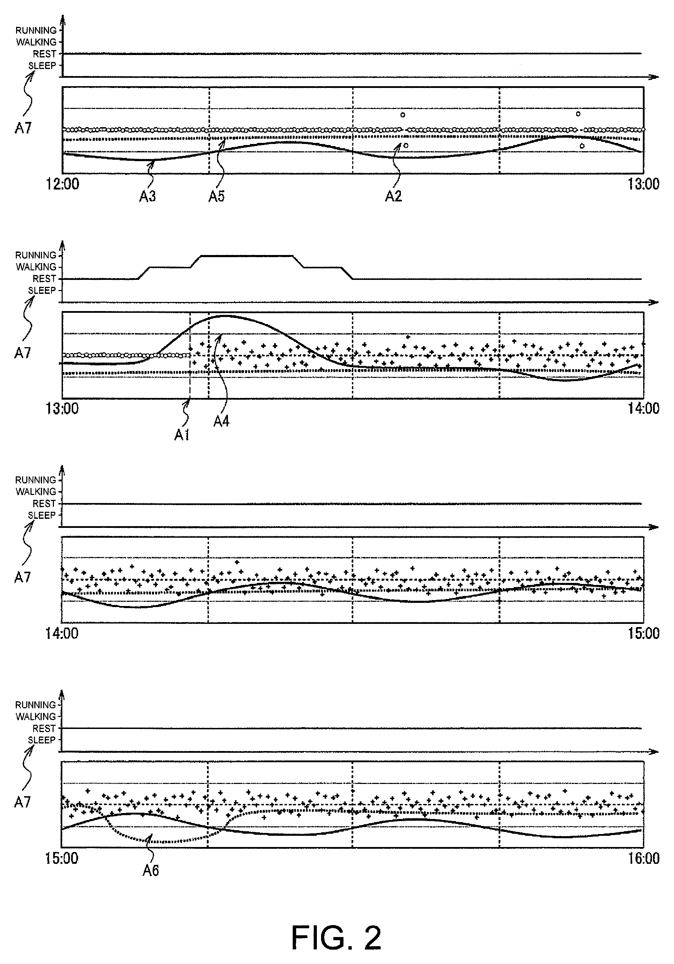

The pulse interval distribution information is information indicating a variation in the pulse interval. The pulse interval distribution information may be information in which a first axis is a time axis and in which a value based on the pulse interval is plotted on a second axis, as described later with reference to FIG. 2.

The processor 230 may also generate information for displaying a reliability index of measurement of the pulse wave information during the measurement period, as the analysis result information.

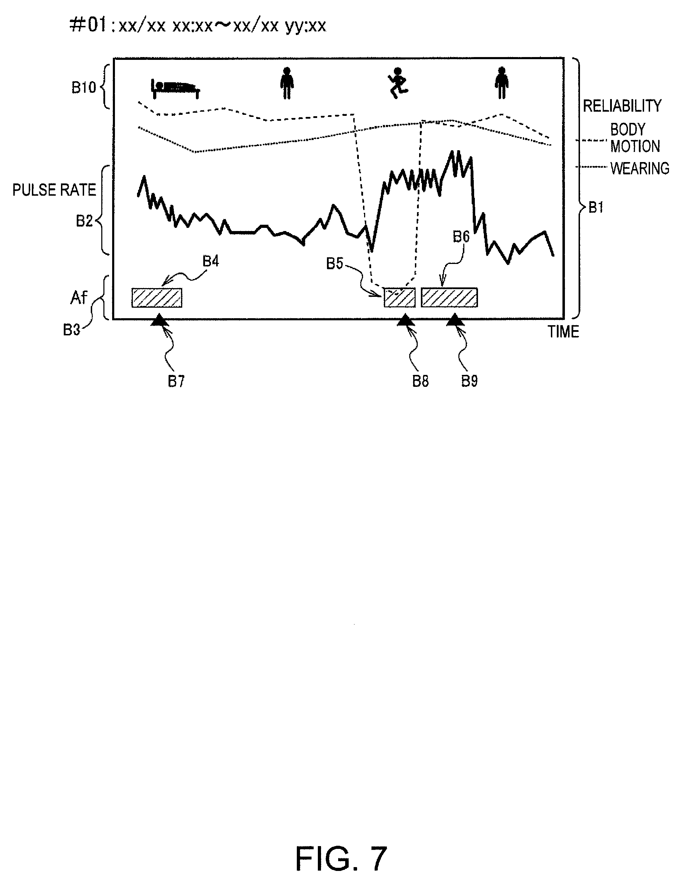

Here, the reliability index is information indicating to what extent the pulse wave measurement is reliable. An example of the reliability index is numerical value information and may be configured in such a way that a greater numerical value indicates that the pulse wave measurement is more reliable (higher reliability), whereas a smaller numerical value indicates that the pulse wave measurement is less reliability (lower reliability), or the other way round. Alternatively, a numerical value closer to a predetermined numerical value may indicate higher reliability. The relation between a specific value of the reliability index and high/low reliability can be implemented with various modifications. For example, in a curve A3 in FIG. 2, described later, since information based on the amount of effective signal of acceleration information is used as a reliability index, a greater numerical value indicates a larger body motion noise and lower reliability. Meanwhile, in FIG. 7, described later, a greater numerical value indicates higher reliability.

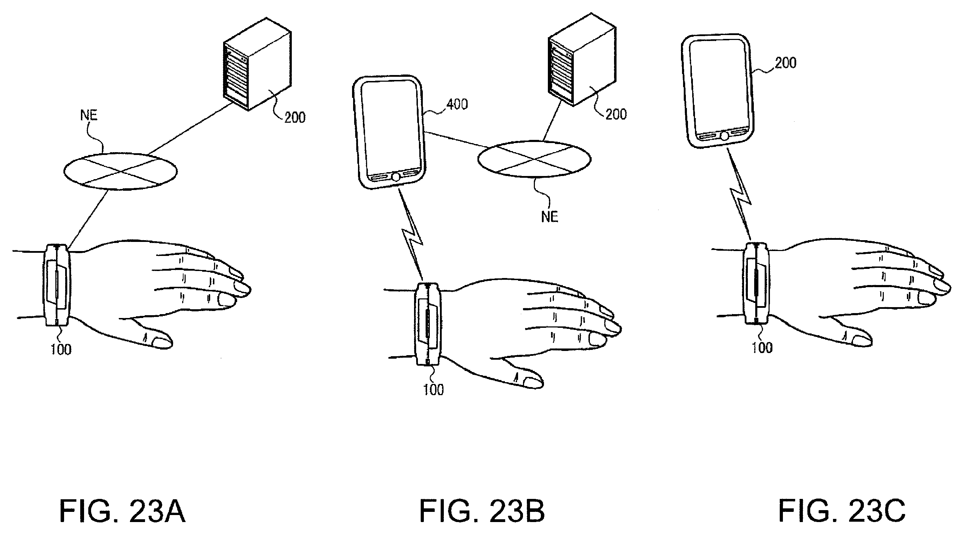

The reliability index in this case is information based on at least one of body motion information of the user and wearing state information of the user. Here, the information based on the body motion information may be the body motion information itself or may be information obtained as a result of performing certain processing on the body motion information. The information based on the wearing state information may be the wearing state information itself or may be information obtained as a result of performing certain processing on the wearing state information. The wearing state information may also be information indicating the wearing state of a measuring device mounted on the user's body. The measuring device in this example is, for example, a pulse wave measuring device 100 as described later with reference to FIGS. 21A to 22. As described later with reference to FIGS. 23A to 23C, the biological information processing system 200 according to the embodiment may be implemented by a device such as a server system or may be implemented by the pulse wave measuring device 100. That is, the measuring device in this example may refer to a device which is different from a device including the biological information processing system 200, or may be refer to a device including the biological information processing system 200.

The processor 230 may generate information for displaying a display object (icon, mark) indicating the pulse wave abnormality period, as the analysis result information.

The processor 230 may also generates information for displaying a pulse wave abnormality period and a period that is not the pulse wave abnormality period in different image display forms, as the analysis result information.

Various specific examples of the different image display forms are possible. For example, different dot shapes may be used, as described with reference to FIG. 2. In FIG. 2, cross-shaped dots are used for a pulse wave abnormality period, and solid white circular dots are used for a period that is not the pulse wave abnormality period. Alternatively, modifications such as changing the color of dots between the pulse wave abnormality period and the other periods, changing the size, or changing the background color, are possible as well. Also, the image display form may be changed by flashing on and off the dots, background or the like in the case of displaying on the display of a predetermined device, though it is practically difficult in the case of printing an analysis report.

The processor 230 may also generate second analysis result information for displaying at least one of a pulse waveform and a pulse interval waveform during a predetermined section in the pulse wave abnormality period.

The use of the pulse wave information causes other problems as well. Specifically, useful information for swift diagnosis needs to be reported efficiently from long-time data. As described above, the pulse wave information is acquired over a period of about three to ten days and therefore results in a massive amount. Therefore, if detailed information, for example, a pulse waveform, which is the waveform of the pulse wave information, is displayed over the entire measurement period (during of test), the doctor has to bear a heavy burden of viewing the information. Therefore, it is preferable that analysis processing on the pulse wave information (for example, determination processing to determine whether an abnormality is seen in the pulse wave information or not) is performed on the side of the biological information processing system and that outline information using the result of the determination is presented.

However, an arrhythmia should be diagnosed by the doctor and it is not desirable to present only the result of the determination by the biological information processing system. In other words, the output (analysis report or the like) from the biological information processing system must also present information that is detailed enough for the doctor to diagnose whether there is an arrhythmia or not. Particularly, the pulse wave information is influenced by the body motion noise or the like and has lower accuracy than in the case of using an electrocardiogram. Therefore, there is a great need for a screen display or the like for checking whether the result of detection of a pulse wave abnormality by the system is appropriate or not.

In short, the biological information processing system needs to generate and output information that satisfies both a first demand that the doctor should avoid viewing all of a massive amount of data and a second demand that information that is detailed enough for the doctor to be able to diagnose an arrhythmia is made available for viewing.

Thus, the applicant proposes a biological information processing system 200 as follows. Specifically, the biological information processing system 200 in the embodiment includes an acquirer 210 which acquires pulse wave information, a processor 230 which performs analysis processing on the pulse wave information to generate analysis result information, and a display processor (output 250) which performs display processing on the analysis result information.

The display processor performs display processing on a first screen where the analysis result information is displayed, and performs the display processing on a second screen where original information used to generate the analysis result information is displayed, when the user (viewer) carries out a predetermined instruction operation.

The arrhythmia in the embodiment is atrial fibrillation. The pulse wave abnormality period may be a period determined as having an occurrence of the arrhythmia, on the basis of the analysis processing on the pulse wave information by the processor 230. In an electrocardiogram, when the heart normally beats once, various characteristic waveforms such as the P-wave, Q-wave, R-wave, and S-wave appear. Therefore, various arrhythmias may be diagnosed by paying attention to differences between such normal waveforms and measured waveforms. Meanwhile, the pulse wave signal is for detecting fluctuations in the blood flow rate due to pulsation. Therefore, a pulse waveform does not have a shape as in the electrocardiogram. Thus, a technique using pulse wave information specializes in processing based on the pulse interval or pulse rate, rather than detecting various arrhythmias, and has a high affinity to detection processing of atrial fibrillation in which the variation in the pulse interval increases at the occurrence of the symptom. Therefore, a case of atrial fibrillation will be described below, as an example. However, as an arrhythmia in the embodiment, employing arrhythmias other than atrial fibrillation (for example, premature contraction or the like) is not precluded.

The first screen is, for example, a screen as shown in FIG. 7 described later. The first screen shown in FIG. 7 is a screen for displaying first analysis result information, similarly described later, and for example, a screen for displaying a pulse rate and body motion information during a pulse wave abnormality period suspected of having an occurrence of an arrhythmia such as atrial fibrillation. A specific example of information displayed on the first screen will be described in detail later.

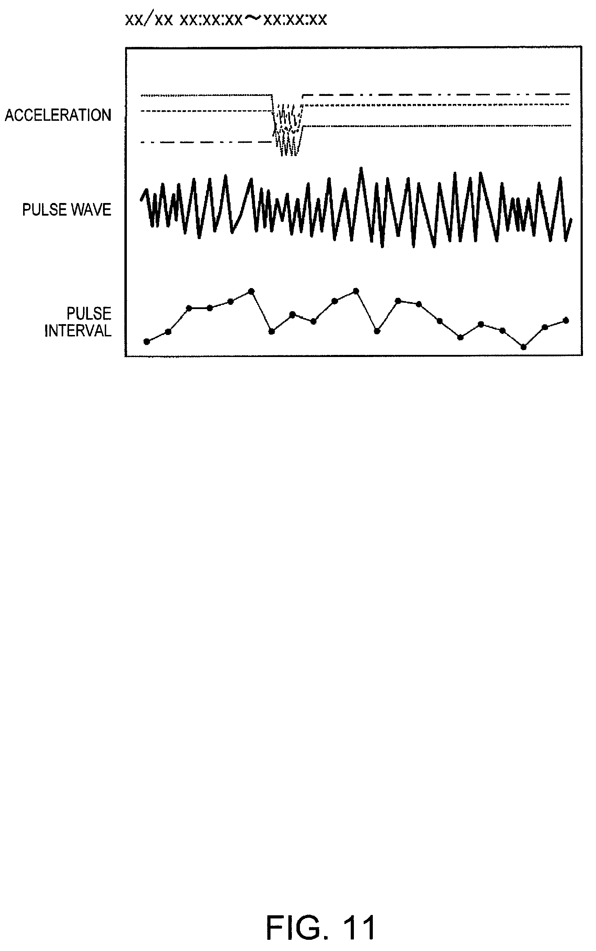

Meanwhile, the second screen is a screen as shown in FIGS. 11 to 13, described later. The second screen shown in FIGS. 11 to 13 is a screen for displaying second analysis result information, similarly described later. The second analysis result information is (a part of) original information used when generating the first analysis result information.

That is, in the embodiment, the doctor or the like, as the viewer, views the first screen and recognizes a pulse wave abnormality period suspected of having an occurrence of atrial fibrillation or the like. Then, the doctor carries out a predetermined instruction operation on the first screen in order to determine whether atrial fibrillation or the like actually occurs during the presented pulse wave abnormality period or not. For example, the doctor carries out a tap operation on image data (pulse waveform) indicating the pulse wave abnormality period on the first screen. Thus, the second screen corresponding to the image data on which the predetermined instruction operation is carried out is displayed. On the second screen, the original information for generating the analysis result information, that is, pulse waveform data, pulse interval information, acceleration signal and the like are displayed, as described above.

In this way, according to the embodiment, it is possible to switch between the analysis result information about the pulse wave information and the original information of the analysis result information, as the information to be displayed. Therefore, since the doctor only has to confirm the information of the pulse waveform or the like that is necessary for diagnosing an arrhythmia, the burden on the doctor can be reduced, compared with the method in which the doctor analyses all of the acquired pulse waveforms and the like.

The display processing may be processing in which image data for displaying the first screen or the second screen is generated, or may be processing in which an instruction to display the first screen or the second screen is given to the display.

The display processor also performs display processing in which a pulse wave abnormality period for determination on an abnormality in the cardiovascular system is identifiably displayed as the analysis result information, for example, on the first screen as shown in FIG. 7, described later. The display processor then performs display processing in which pulse interval information is displayed as the original information, for example, on the second screen as shown in FIG. 11, described later.

Thus, the viewer can view, on the second screen, the pulse interval information corresponding to the pulse wave abnormality period displayed on the first screen, or the like.

Here, the abnormality in the cardiovascular system is, for example, an arrhythmia. Therefore, the viewer can the pulse wave abnormality period during which an arrhythmia occurs in the test subject and the pulse interval information during the pulse wave abnormality period, or the like.

The display processor may also perform display processing in which pulse rate information is displayed on the first screen as the analysis result information, and may perform display processing in which pulse waveform information is displayed on the second screen as the original information.

Thus, the viewer can view, on the second screen, the pulse waveform information corresponding to the pulse rate information displayed on the first screen, or the like.

Also, compared with measurement based on an electrocardiogram, measurement of pulse wave information is more affected by noises such as a body motion noise. Specifically, in the analysis of a photoelectric pulse wave signal, though the pulse wave analysis can be corrected by various kinds of noise elimination processing in the phase where the analysis is affected by a body motion noise, there can be no complete correction processing and the reliability of the result of the analysis of the pulse wave signal during that period drops. Also, in the phase of wearing failure, the amplitude of the pulse wave signal drops and the reliability equally drops.

Therefore, in the biological information processing system 200, even when the pulse wave information is determines as abnormal (a pulse wave abnormality period is detected), the information can be wrong. From the perspective of the doctor, even if the pulse wave abnormality period is identifiable on the basis of the analysis result information, it is necessary to carefully examine whether the abnormality appears in the pulse wave information as a result of the user having an arrhythmia or as a result of the influence of a noise while the user is normal, and this causes a heavy burden.

Thus, the display processor performs display processing in which a reliability index of pulse wave measurement found on the basis of body motion information of the user is displayed on the first screen as the analysis result information, and performs display processing in which the body motion information is displayed on the second screen as the original information.

Thus, if a plurality of pulse wave abnormality periods is displayed via the analysis result information, a period with higher reliability, that is, a period during which not a noise but the occurrence of an abnormality in the user is suspected can be used preferentially for diagnosis, and the burden of diagnosis can be reduced.

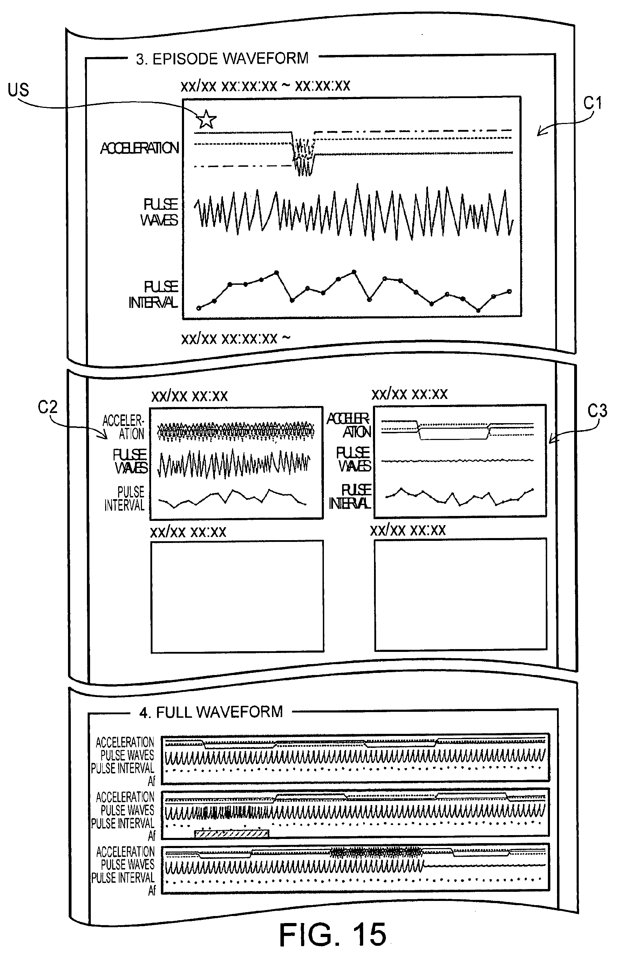

In the description below, an embodiment using the reliability index in a displayed form will be described. However, the reliability index need not necessarily be displayed and may be internally used by the biological information processing system 200. In such a case, information with higher reliability may be preferentially used at the time of displaying detailed information as the original information of the analysis result information. Thus, the doctor can preferentially view the information with high reliability, without having the reliability index itself displayed. Of course, the reliability index may be displayed and the information with high reliability may be preferentially displayed, as described later with reference to FIG. 15 and the like.

2. Specific Example of Analysis Result Information

Details of the analysis result information in the embodiment will be described. Hereinafter, a display screen based on the analysis result information will be described as an example. However, the analysis result information may be screen information itself or may be other types of information that can generate screen information. Also, the screen described below is not limited to the display of an electronic apparatus or the like, and may be a printout on a paper medium or the like.

In the following description, it is assumed that, prior to the preparation of the analysis result information, information necessary for display is acquired from among the pulse wave information, the body motion information and each type of information described above, and that, on the basis of these, the pulse interval is found at a first rate (for example, once every second), and the result of determination on whether it is a pulse wave abnormality period or not, and the reliability index of pulse wave measurement are found at a second rate (for example, once every 20 seconds, or it may be the same as the first rate in some cases). Details of processing to find each type of information will be described later, along with an example of system configuration.

2.1 Example of Screen for Displaying Action Information

For example, if the pulse waveform or the pulse interval waveform is displayed over the entire measurement period, as shown in the full waveform of FIG. 15, described later, it is possible to display, without exception, information corresponding to the state where atrial fibrillation is occurring. However, FIG. 15 shows an only a part of the full waveform, and viewing all the data taken over three to ten days puts a heavy burden on the doctor. Even if the doctor tries to extract and focus on a part of the information, with only the full waveform displayed, there is few clues to which part should be extracted and the doctor may end up having to check all the data.

That is, the analysis result information in the embodiment needs to show data over a relatively long period (in a narrow sense, the entire measurement period) in a form that enables understanding at a glance and to intelligibly present to the doctor a part to be a high-priority target of viewing. Therefore, in the embodiment, the first analysis result information for identifiably displaying a pulse wave abnormality period in the measurement period is generated, as described above.

A specific example of a display screen displayed using the first analysis result information is shown in FIG. 2. FIG. 2 is also a specific example of a display screen for identifiably displaying a pulse wave abnormality period for determination on an arrhythmia and for displaying action information of the user. In FIG. 2, the information is shown as divided in four rows. In each row, the horizontal axis represents time. For example, 12:00 in the top row indicates 12:00 (12 o'clock) on a predetermined day (or 12 hours 00 minutes past the start of the measurement period). That is, each row in FIG. 2 shows data corresponding to one hour, and information corresponding to a relatively long period of four hours can be displayed in the entirety of FIG. 2.

In FIG. 2, changes in time series in the pulse interval are plotted as dots in the bottom of each row. Specifically, the vertical axis in FIG. 2 may logarithmically express the proportion of the value of the pulse interval at a predetermined timing to the value of the pulse interval at the immediately preceding timing. That is, if a dot is plotted at the position of 0 on the vertical axis (in FIG. 2, the center position on the vertical axis), it means that the pulse interval at this timing is the same as the pulse interval at the previous timing. That is, in this graph, as dots are plotted at positions closer to 0, it means a smaller fluctuation in the pulse interval, whereas a greater variation in plotted positions indicates a greater fluctuation in the pulse interval. That is, this shows pulse interval distribution information.

Here, the term "plot" corresponds to processing in which, in the case where information prescribed by a set of a plurality of values and a space where one value corresponds to one dot (position) are provided, each value included in the set is arranged (displayed) in association with the space. In the example of FIG. 2, a set of proportion information of the pulse interval, which is one-dimensional values (scalar) acquired in time series, is arranged at corresponding positions in the direction of the vertical axis. Alternatively, it is also possible to consider that FIG. 2 is equivalent to processing in which a set of two-dimensional values (two-dimensional vectors) made up of proportion information and acquisition time is arranged in a two-dimensional space (plane) made up of a time axis and a proportion information axis.

In the example of FIG. 2, the distribution information of the pulse interval is information in which a first axis is the time axis and in which a value based on the pulse interval is plotted on a second axis. In the example of FIG. 2, the first axis is the horizontal axis and the second axis is the vertical axis. The value based on the pulse interval is a logarithm of the proportion to the pulse interval at the immediately preceding timing, as described above.

As the proportion to the immediately preceding timing is thus used, plotted dots concentrate around the position of a predetermined value (in this example, 0) if the variation is small, whereas plotted dots are dispersed in a broad range if the variation is large. That is, since changes in time series in the concentration and dispersion of plotted dots can be displayed, it is possible to make it easier for the viewer to diagnose the arrhythmia.

Here, the value of the pulse interval has the characteristic of fluctuating according to the state of the user (patient). For example, when the user is in a sleeping state or in a resting state, the pulse interval is long, whereas when the user is in an exercising state, the pulse interval is short. Even if the value of the pulse interval changes because of a shift from the sleeping state to the exercising state, the change is a normal reaction and not due to the arrhythmia. That is, the variation in the pulse interval can include variation due to a change in the state of the user (unrelated to the arrhythmia) and variation due to the arrhythmia. If the value of the pulse interval itself is plotted, the variation due to both factors is displayed. However, if the proportion to the immediately preceding timing is displayed, the influence of the variation due to the change in the state of the user, which is a longer-term change, can be restrained.

In view of this point, in the example of FIG. 2, the distribution information of the pulse interval is defined as information in which the first axis is the time axis and in which the proportion information of the pulse interval at a second timing to the pulse interval at a first timing is plotted on the second axis. However, the value on the second axis is not limited to the proportion information, since it suffices that the influence of the variation due to the change in the state of the user can be restrained. For example, the distribution information of the pulse interval may be information in which the first axis is the time axis and in which difference information of the pulse interval at a second timing from the pulse interval at a first timing is plotted on the second axis. The proportion information and the difference information may be the proportion itself or the difference itself, or may be information found from the proportion or the difference, or may be information corresponding to the proportion or the difference.

Each of the first timing and the second timing is an acquisition (calculation) timing of the pulse interval or a timing decided on the basis of the acquisition timing. As an example, a timing (for example, a timing indicating the end point), of a pulse interval calculation section, described later, may be employed. Since the proportion information and the difference information are used in view of intelligibly presenting the degree of fluctuation in the pulse interval as described above, the first timing and the second timing, in a narrow sense, are next to each other.

In the example of FIG. 2, before a timing indicated by A1, plotted dots are stable at positions substantially close to 0. Therefore, by viewing this information, the doctor can diagnose that the arrhythmia does not occur during this period. Alternatively, in the determination by a determination index calculator 224, described later, it is determined that this period is not a pulse wave abnormality period. Although there are dots where there is a large fluctuation in the pulse interval from the immediately preceding timing, as indicated by A2 or the like, a variation to this extent can be seen in normal users and therefore poses no problem.

Meanwhile, after the timing indicated by A1, plotted dots are broadly dispersed and no regularity can be seen in the dispersion. Therefore, the doctor suspects the occurrence of the arrhythmia during this period. Alternatively, in the determination by the determination index calculator 224, described later, this period is determined as a pulse wave abnormality period.

Up to this point, the technique using the proportion information or the like, in order to restrain the influence of the fluctuation in the pulse interval unrelated to the arrhythmia, has been described. However, since the fluctuation in the pulse interval unrelated to the arrhythmia is similarly important information indicating the state of the pulse of the user, it is useful to present this information to the doctor. That is, a form of embodiment in which both the fluctuation in the pulse interval due to the change in the state of the user (unrelated to the arrhythmia) and the variation in the pulse interval due to the arrhythmia are displayed is highly conceivable.

The fact that the influence of the fluctuation in the pulse interval unrelated to the arrhythmia can be restrained by using the proportion information or the difference information of the pulse interval means, in other words, that when the proportion information or the like is used, the fluctuation in the pulse interval due to the change in the state of the user is less likely to appear in the display. That is, if even the fluctuation in the pulse interval due to the change in the state of the user is to be displayed while using the proportion information or the like, it is desirable to separately display a change in time series in the pulse rate or the pulse interval.

Alternatively, the value of the pulse interval itself may be used as the value based on the pulse interval. As described above, the value of the pulse interval itself includes both the fluctuation due to the change in the state of the user and the variation due to the arrhythmia. Therefore, by using the value of the pulse interval, it is possible to express both at the same time. That is, the "value based on the pulse interval" used for displaying the distribution information of the pulse interval may be the value of the pulse interval itself or may be the proportion information or the difference information. Various modifications are possible.

By thus employing the display shown in FIG. 2, the viewer can presume whether atrial fibrillation has occurred or not, on the basis of the degree of dispersion of plotted dots. However, in the embodiment, the biological information processing system 200 may determine whether it is a pulse wave abnormality period or not, and the result of the determination may be displayed in an intelligible (highly visible) form.

Specifically, the processor 230 generates information for displaying a pulse wave abnormality period and a period that is not the pulse wave abnormality period in different image display forms, as the analysis result information. Various specific examples of the different image display forms are possible. For example, different dot shapes may be used, as shown in FIG. 2. In FIG. 2, cross-shaped dots are used for a pulse wave abnormality period, and solid white circular dots are used for a period that is not the pulse wave abnormality period.

By thus changing the image display form between a pulse wave abnormality period and a period that is not the pulse wave abnormality period, whether it is a pulse wave abnormality period or not can be easily discriminated on the basis of the image display form (in this example, dot shape), and the burden on the doctor can be reduced. In the example of FIG. 2, it can be readily understood that there is a possibility of atrial fibrillation occurring after the timing of A1 and at least until 16:00. Therefore, the doctor can make a decision to view detailed data corresponding to this period. As the change of the image display form, modifications such as changing the color of dots between the pulse wave abnormality period and the other periods, changing the size, or changing the background color, are possible as well. Also, the image display form may be changed by flashing on and off the dots, background or the like in the case of displaying on the display of a predetermined device, though it is practically difficult in the case of printing an analysis report.

In FIG. 2, changes in time series in the action information of the user are displayed at the top (A7) of each row. The action information in this example is activity type information indicating which state the user is in, of a sleeping state, a resting state, a walking state, and a running state. The amount of activity in each state is expected to be in the relation of sleeping state < resting state < walking state < running state. Therefore, by arranging the respective states in the direction of the vertical axis in order of the amount of activity corresponding to each type of activity, as shown in FIG. 2, the action information of FIG. 2 also has the aspect of amount of activity information.

The action information (activity type information) in this example can be found, for example, on the basis of the pulse wave information. Specifically, since it is known that the pulse rate rises as the activity of the user increases, the type of activity can be determined on the basis of comparison processing between the pulse rate and a predetermined threshold, or the like. As an example, if the pulse rate is equal to or below a first threshold, it may be determined that the user is in the sleeping state, whereas if the pulse rate is above the first threshold and equal to or below a second threshold, it may be determined that the user is in the resting state. The resting state in this example refers to a state where the user is awake with little body motion. Therefore, when the body motion information such as acceleration information is used, it may be difficult to discriminate the resting state from the sleeping state. In this respect, the pulse wave information reflects internal activity of the user that is invisible from outside and mental activity as well, and therefore enables accurate determination on the sleeping state and the resting state. Also, autonomic nerve activity information such as LF and HF, described later, may be used for determination on the type of activity. Alternatively, both of the pulse wave information and the body motion information may be used to determine the type of activity. The type of activity may also be classified roughly, such as an exercising state, an everyday life state, and a sleeping state. For the determination on the action of the user, various techniques are known, including a technique using pulse wave information. In the embodiment, these techniques can be applied broadly.

Thus, it is possible to display the result of detection of a pulse wave abnormality period and the action information in association with each other. For example, if an arrhythmia occurs during a pulse wave abnormality period, it is possible to specify whether the arrhythmia is sympathetic nerve-dependent or parasympathetic nerve-dependent. In the sleeping state, parasympathetic nerves are dominant. Therefore, if it is determined that the user is in the sleeping state during (or before and after) a pulse wave abnormality period, it can be presumed that the arrhythmia is parasympathetic nerve-dependent. Meanwhile, in the awake state, sympathetic nerves are dominant. Therefore, if it is determined that the user is in the awake state during (or before and after) a pulse wave abnormality period, it can be presumed that the arrhythmia is sympathetic nerve-dependent.

In the example of FIG. 2, since the pulse wave abnormality period in this range corresponds to the awake state, it can be presumed that the arrhythmia is sympathetic nerve-dependent. If information corresponding to a longer period is viewed and it is found out, for example, that other pulse wave abnormality periods correspond to the awake state as well and that no pulse wave abnormality period corresponding to the sleeping state is observed (or pulse wave abnormality periods corresponding to the sleeping state are fewer or shorter than the pulse wave abnormality periods corresponding to the awake state), it is possible to determined that the arrhythmia is highly likely to be sympathetic nerve-dependent.

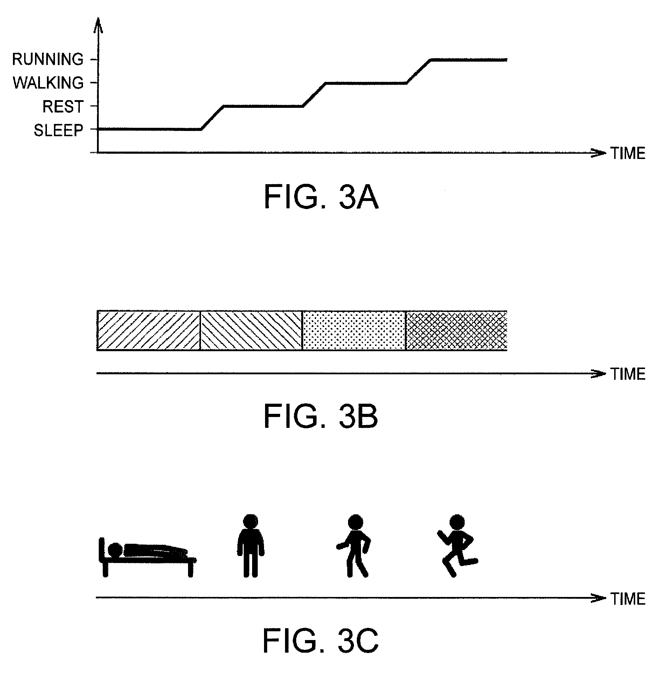

In A7 in FIG. 2, a graph with the horizontal axis representing type of activity and the horizontal axis representing time is used, as shown in FIG. 3A. However, the display of the type of activity is not limited to this example. For instance, a bar-shaped display object may be displayed along the time axis, and the color of (or the pattern in) the bar may be changed according to the type of activity, as shown in FIG. 3B. Alternatively, icons indicating types of activity may be displayed along the time axis, as shown in FIG. 3C. In this case, for example, the display of FIG. 3B or FIG. 3C, instead of FIG. 3A, may be shown at the position indicated by A7 in FIG. 2. Also, the display of the action information can be implemented with modifications other than FIGS. 3A to 3C, such as changing the type of line or the color of line in the graph according to the type of activity.

Also, as indicated by A3 and A5 in FIG. 2, the reliability index may be displayed along with the pulse wave abnormality period which is identifiably displayed. The reliability index in this case is information based on at least one of the body motion information of the user and the wearing state information of the user. Here, the information based on the body motion information may be the body motion information itself or may be information obtained as a result of performing certain processing on the body motion information. The information based on the wearing state information may be the wearing state information itself or may be information obtained as a result of performing certain processing on the wearing state information. The wearing state information may also be information indicating the wearing state of a measuring device mounted on the user's body. The measuring device in this example is, for example, a pulse wave measuring device 100 as described later with reference to FIGS. 21A to 22. As described later with reference to FIGS. 23A to 23C, the biological information processing system 200 according to the embodiment may be implemented by a device such as a server system or may be implemented by the pulse wave measuring device 100. That is, the measuring device in this example may refer to a device which is different from a device including the biological information processing system 200, or may be refer to a device including the biological information processing system 200.

A3 in FIG. 2 indicates a curve showing changes in time series in the reliability index related to body motion noise. The vertical axis represents the magnitude of the body motion noise. Here, the body motion noise is higher as it goes upward on the vertical axis, and lower as it goes downward on the vertical axis. Specifically, an amount of effective signal of the body motion information or the like may be used as the reliability index.

Thus, it is possible to intelligibly present that if the value on the curve A3 is large (situated in an upper part on the vertical axis), the body motion noise is high and the reliability of measurement of pulse wave information is low. Therefore, while the biological information processing system 200 determines that the period indicated by A4 is a pulse wave abnormality period, the doctor can be allowed to make a decision that the possibility that the variation in the pulse interval during this period may be due to the body motion noise cannot be eliminated. Displaying the reliability index enables the doctor to easily determine whether large variation in the distribution information of the pulse interval is due to an arrhythmia or due to a noise, when it happens. Also, it is possible to differentiate pulse wave abnormality periods according to the reliability, instead of treating all pulse wave abnormality periods equally, and to reduce the burden on the doctor further.

A5 in FIG. 2 indicates information based on the wearing state information. Specifically, the vertical axis may represent the effective value of the pulse wave signal (effective value of the amplitude of the pulse waveform). Thus, it is possible to intelligibly present that if the value on the curve A5 is small (situated in a lower part of the vertical axis), the signal level is low, the wearing state is not good, and the reliability of measurement of the pulse wave information is low. Specifically, it is possible to easily present to the doctor that the reliability during a period A6 is low, as in the period A4.

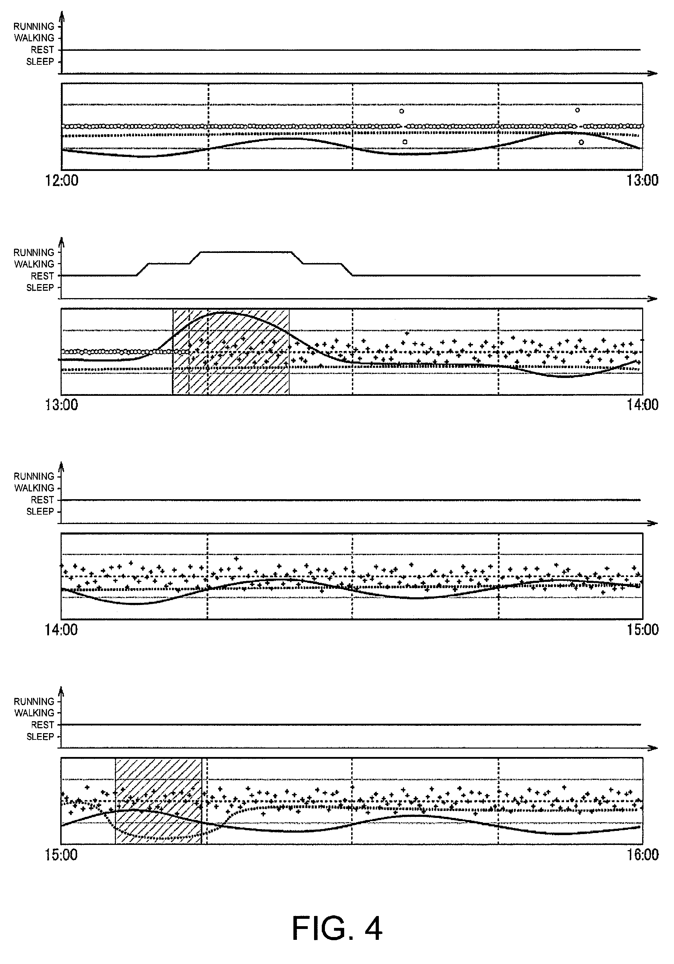

For a period which is determined as a pulse wave abnormality period with low reliability, such as A4 and A6, further highlighting may be carried out to discriminate this period from periods with high reliability. As an example, the background color in the section with lower reliability may be changed, as shown in FIG. 4. Thus, it is visually clarified that the determination based on the pulse wave information is wrong with respect to the section where the background color is changed. Therefore, the doctor only has to make a diagnosis, focusing on the section where the background color is not changed (or the section where the background color indicates high reliability). Thus, the burden of viewing information can be reduced.

Thus, if a plurality of pulse wave abnormality periods is displayed via the analysis result information, a period with higher reliability, that is, a period during which not a noise but the occurrence of an abnormality in the user is suspected can be used preferentially for diagnosis, and the burden of diagnosis can be reduced.

2. 2 Modification 1 (Amount of Activity Information, Psychological State Information, External Environment Information, Location Information)

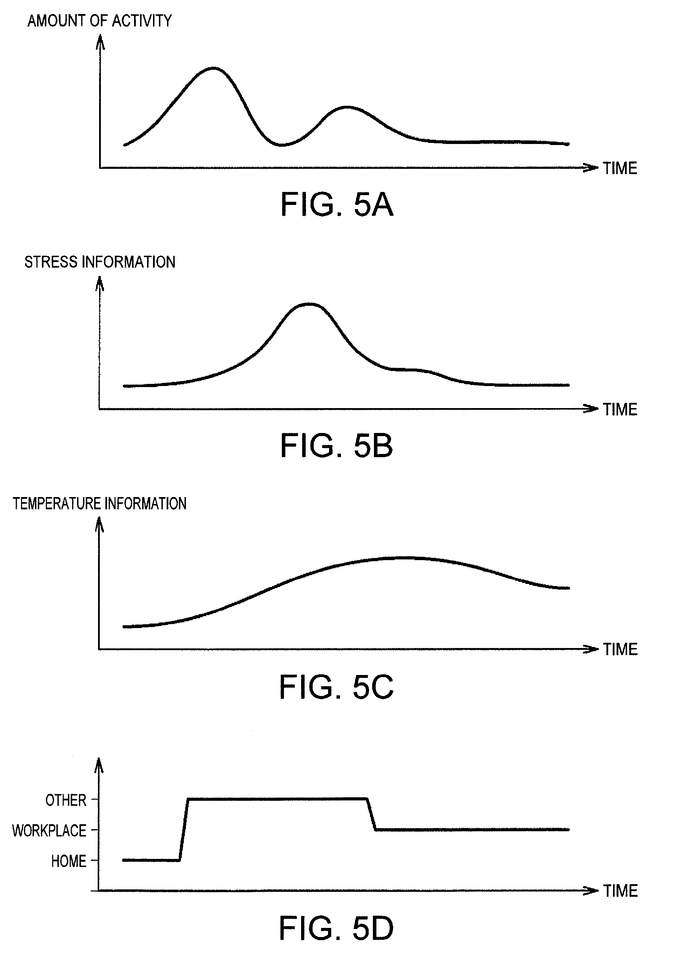

As the information displayed along with the result of determination on the pulse wave abnormality period, various kinds of information are conceivable, as described above. For example, the amount of activity information may be displayed as the action information, as shown in FIG. 5A. The amount of activity information is information such as calories burned, oxygen intake, or METs, as described above, and can be quantitatively handled (expressed by numerical values). In FIG. 5A, the horizontal axis is a time axis, and the vertical axis is an axis representing the amount of activity. By changing the graph of action information shown in A7 of FIG. 2 to FIG. 5A, it is possible to intelligibly display the relation between the pulse wave abnormality period and the amount of activity.

Oxygen intake VO2 can be measured, for example, by using a breath measuring device. Since it is known that oxygen intake can be used as an index value indicating exercise intensity, oxygen intake itself can be used as the amount of activity information. Also, since a method of finding calories burned from oxygen intake is known, the resulting calories burned may be used as the amount of activity information. However, since a breath measuring device is often large in size, the amount of activity information may be found using the pulse wave information. Specifically, it is known that oxygen intake VO2 and heart rate HR are correlated with each other. For example, the relation of the following equation (1) is known, where maximum VO2 is VO2m, VO2 at rest is VO2r, heart rate at rest is HRr, and maximum heart rate is HRm. However, the left side of the equation (1) uses minute oxygen intake.

$$\frac{\dot{V}O_2 - \dot{V}O_{2r}}{\dot{V}O_{2m} - \dot{V}O_{2r}} \times 100(\%) = \frac{(HR - HR_r)}{(HR_m - HR_r)} \times 100(\%) \quad \cdots \quad (1)$$

That is, if a pulse rate can be found from the pulse wave information, since the pulse rate corresponds to (coincides with) the heart rate HR in the case where the user has no abnormality, the oxygen intake VO2 can be found by the equation (1). Therefore, it is possible to find oxygen intake or calories burned from the pulse rate and use the oxygen intake or calories burned as the amount of activity information.

Also, the psychological state information may be displayed, specifically, stress information may be displayed, as shown in FIG. 5B. In FIG. 5B, the horizontal axis is a time axis, and the vertical axis is an axis representing intensity of stress. For example, it is known that the numerical value of pulse rate rises due to mental stress. Therefore, it is possible to estimate the intensity of stress on the basis of the degree of rise in the pulse rate compared with a reference pulse rate (pulse rate at rest, minimum pulse rate). Moreover, various methods of finding a stress index from the pulse wave information or the like are known. In the embodiment, these methods can be applied broadly.

By changing A7 in FIG. 2 to FIG. 5B, it is possible to intelligibly display the relation between the pulse wave abnormality period and the stress information. If a correlation between a period of high stress and a pulse wave abnormality period is observed, it can be presumed that an arrhythmia corresponding to the pulse wave abnormality period is induced by stress.

Also, the external environment information may be displayed along with the result of determination on the pulse wave abnormality period. FIG. 5C shows an example of screen for displaying the temperature information. The temperature information in this example may be temperature difference information between the body surface temperature and the ambient temperature, as described above.

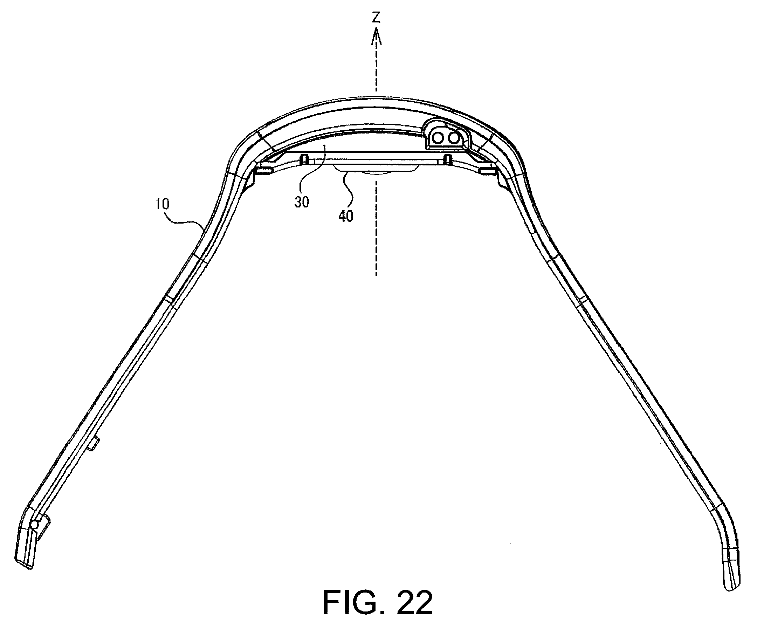

Specifically, the pulse wave measuring device may measure temperature at two points at different distances from the body surface of the user. For example, in the example of FIG. 22, described later, a plurality of temperature sensors may be provided at different positions in an Z-axis direction intersecting with a case 30 (in a direction perpendicular to the face of a wristwatch, or the like). In this case, in the positive Z-axis direction in FIG. 22, the body surface of the user, a first temperature sensor, a second temperature sensor, and a boundary surface between the pulse wave measuring device and the outside are situated in this order. In this case, in the Z-axis direction, there is a temperature gradient between the body surface temperature T0 of the user and the ambient temperature (in a narrow sense, temperature on the boundary surface) T3, and the outputs from the first and second temperature sensors are the temperatures T1 and T2 corresponding to the positions where the temperature sensors are arranged, along the temperature gradient (positions on the Z-axis, and in this example, z1 and z2), as shown in FIG. 6. That is, by estimating the temperature gradient in the Z-axis direction from T1 and T2 when these are detected (on the assumption of linearity in FIG. 6), it is possible to estimate the body surface temperature T0 and the ambient temperature T3. The temperature gradient may be found using a heat balance model or the like. The format is not limited to a linear function and various modifications are possible. The temperature difference information is, for example, T0-T3, but is not limited to this.

In view of the load on the human body, while an environment where the ambient temperature is simply too high or too low can be a load factor, a sudden change in the ambient temperature may be a greater load factor. For example, a case is known where a cerebral hemorrhage occurs immediately after the person goes outdoors from a heated room during a cold season in winter. Such a circumstance also causes a heavy load on the heart, and a sudden temperature change can be a factor in an arrhythmia. When the ambient temperature suddenly changes, the difference from the body surface temperature is considered to be greater than when the ambient temperature changes gently (or when a certain time has passed from the change). That is, by using the temperature difference information from the body surface temperature, rather than using the ambient temperature itself, it is possible to properly show the load of temperature on the user, and this is effective for determination on whether the arrhythmia is induced by temperature or not.

Temperature is not the only factor. There is an environment where a person feels comfortable and an environment where the person feels uncomfortable. While criteria for this vary from one person to another, an environment where a user feels uncomfortable causes a load on the user anyway and therefore can be a factor in an arrhythmia. It is also conceivable that a specific environment can induce an arrhythmia, regardless of the user feeling comfortable or uncomfortable. For example, an occurrence of an arrhythmia can be observed under a specific temperature condition, even if the user does not feel uncomfortable. Therefore, by acquiring external environment information indicating the external environment, and then examining the correlation between the external environment and the pulse wave abnormality period, it is possible to specify whether the arrhythmia is induced by the external environment or not, and if it is induced by the external environment, specifically what environment is the factor. Thus, it is possible to take measures such as avoiding the specific external environment so as to restrain the occurrence of an arrhythmia.

Since FIG. 5C shows an example using temperature information, the horizontal axis is a time axis, and the vertical axis is an axis representing temperature (temperature difference, for example, in °C). However, other types of information can be similarly displayed. For example, in the case of barometric pressure information, the vertical axis may be changed to barometric pressure (for example, in Pa). In the case of external light information, the vertical axis may be changed to illuminance (for example, in lux). If low barometric pressure is considered to cause a load on the user, an index value indicating this load, for example, an index value which increases as barometric pressure drops, may be found, and this index value may be taken on the vertical axis. Also, if flashing on and off of external light is considered to cause a load on the user, an index value indicating this load, for example, an index value which increases as the number of times the flashing takes place increases or as the frequency of flashing increases, may be found, and this index value may be taken on the vertical axis. In the case of ambient sound information, the vertical axis may be changed to sound volume (for example, in dB). In this case, too, if a sound in a specific frequency band poses a problem, various modifications are possible, such as finding only the sound volume in this frequency band and displaying this sound volume on the vertical axis.

As shown in FIG. 5D, the location information may be displayed along with the result of determination on the pulse wave abnormality period. In FIG. 5D, the vertical axis simply represents attributes of locations, instead of the magnitude of a quantitative index value. In other words, it is not the relation in which the load on the user increases as it goes upward on the vertical axis.

Various methods for specifying the current location of the user are known, such as using the GPS (global positioning system) or using base station information of mobile phone, and location information can be acquired using latitude, longitude and the like. Therefore, by associating a specific location (latitude and longitude) with what attribute the location has, it is possible to specify the location of the user. For example, with commonly used map information, what kind of building or facility there is at which location can be seen. Therefore, the location of the user may be specified on the basis of such information and the measured location information (latitude and longitude). Since locations such as "home" and "workplace" vary from one user to another, the user may be made to input the location by using a certain interface.

Thus, the occurrence of a pulse wave abnormality period and the location of the user at the time can be associated with each other. If there is a correlation indicating that a pulse wave abnormality period tends to occur in a specific place, measures can be taken such as avoiding to that place. As an example, if a pulse wave abnormality period tends to be detected when the user is at the workplace, it is possible to determine that measures such as taking temporary leave from work can be effective, or the like.

As for the display of the location information, various modifications are possible. For example, the location information may be shown using a two-dimensional image such as the one used to display a map or the like. Specifically, a map image showing the location at every predetermined time interval may be prepared and displayed in the area indicated by A7 in FIG. 2. Alternatively, a map image showing the location at the timing when a pulse wave abnormality period is detected may be prepared and displayed in one of the areas in FIG. 2 in such a way that its relation with the pulse wave abnormality period can be seen.

As described above, as the information displayed along with the information for identifiably displaying the pulse wave abnormality period, any of the information shown in FIGS. 3A to 3C, FIGS. 5A to 5D or their modifications, may be used. The displayed information is not limited one type, and a plurality of the kinds of information shown in FIGS. 3A to 3C, FIGS. 5A to 5D or their modifications, can also be displayed.

2.3 Modification 2 (Another Example of Identifiably Displaying Pulse Wave Abnormality Period)

The information displayed along with the information shown in FIGS. 3A to 3C or FIGS. 5A to 5D may be any information which identifiably displays the pulse wave abnormality period, and the display form is not limited to FIG. 2. For example, the processor 230 may generate information for displaying a display object (icon, mark) indicating the pulse wave abnormality period, as the first analysis result information. The analysis result information according to the embodiment may be information for displaying the screen shown in FIG. 7.

In FIG. 7, the horizontal axis represents time. In an area B1, changes in time series in the reliability index are displayed. In an area B2, changes in time series in the pulse rate are displayed. Also, in an area B3, a display object (in the example of FIG. 7, a bar) is displayed in a range corresponding to a period determined as a pulse wave abnormality period. In an area B10, the action information (activity type information, in this example, the icon shown in FIG. 3C) is displayed.

In the example of FIG. 7, it is shown that three pulse wave abnormality periods B4 to B6 indicated by bars are detected in a display target period (for example, a predetermined day of the measurement period). The viewer can easily understand in which period the pulse wave abnormality periods are detected, on the basis of the presence/absence of bars and the positions of the bars on the horizontal axis. That is, the pulse wave abnormality period and the other periods can be displayed in a visually recognizable manner on the basis of the display object, using the presence/absence, shape, color and the like of the display object.

In the example of FIG. 7, when displaying the reliability index, it is assumed that the reliability is higher as it goes upward on the vertical axis, and lower as it goes downward. That is, in the case of the reliability index based on the body motion noise, the reliability index is situated downward as the amount of body motion noise (for example, the amount of effective signal of acceleration information) becomes greater. In this respect, the example of FIG. 7 is different from the display of the reliability index in FIG. 2. In this way, the method for displaying the reliability index can be implemented with various modifications.

In FIG. 7, a pulse rate waveform which shows changes in time series in the pulse rate is displayed in addition to the display object (icon, mark) indicating the pulse wave abnormality period. Also, the pulse rate waveform can be changed to a pulse interval waveform.

Thus, it is possible to display more detailed information based on the pulse wave information as well as the result of determination on the pulse wave abnormality period. Therefore, it is possible to present, to the doctor, information for diagnosing whether an arrhythmia truly occurs during the pulse wave abnormality period or not.

Since the occurrence of atrial fibrillation may continue for several tens of minutes in some cases, one pulse wave abnormality period may have a length of several tens of minutes as well. As described later, the second analysis result information displays information corresponding to at least a section (for example, a predetermined 20-second or 1-minute section) in a pulse wave abnormality period. Therefore, in the case of displaying the information of one pulse wave abnormality period with the use of the second analysis result information, information immediately after the start of this pulse wave abnormality period may be displayed, or information immediately before the end of the pulse wave abnormality period may be displayed. Also, information corresponding to an intermediate timing may be displayed.

Thus, since a section displayed using the second analysis result information can be freely set, which section in the pulse wave abnormality period is used for the display via the second analysis result information may be shown to the viewer. This enables the doctor to understand which section in the pulse wave abnormality period the data currently viewed corresponds to, when viewing the second analysis result information. For example, triangular objects as indicated by B7 to B9 in FIG. 7 may be shown. In the case of FIG. 7, it is shown that information during the sections corresponding to the vertices of the triangular objects, of the pulse wave abnormality periods, is displayed via the second analysis result information.

As described above, the processor 230 may perform determination processing on the pulse wave abnormality period on the basis of the pulse interval and generate the first analysis result information on the basis of the result of the determination processing with respect to each section in the measurement period and the reliability index for each section. Thus, the result of the determination processing (in FIGS. 2 and 4, the shape of plotted dots, and in FIG. 7, the presence/absence of a bar), and the reliability index (in FIGS. 2 and 4, A3 and A5, and in FIG. 7, the area B1) can be displayed together and therefore it is possible to show a display that reduces the burden of viewing on the doctor.

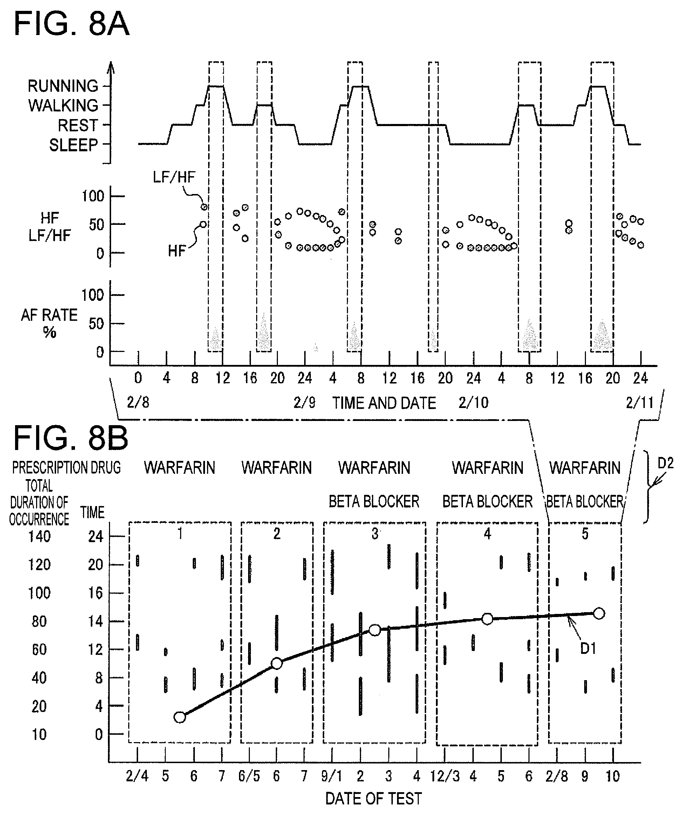

Another modification of the information which identifiably displays the pulse wave abnormality period is possible. FIGS. 8A and 8B show an example of a display screen when the analysis result information is displayed. FIG. 8A corresponds to information generated on the basis of the results of one round of test. FIG. 8B corresponds to information generated on the basis of the results of a plurality of rounds of tests. That is, the processor 230 may generate first information corresponding to the result of analysis on pulse wave information acquired during a first period, and second information corresponding to the result of analysis on pulse wave information acquired during a longer period than the first period. The output 250 may output the first information and the second information together.

The first period in this example refers to, in a narrow sense, a period during which one round of test is conducted. The second period refers to a period during which a plurality of rounds of tests is conducted. Also, the term "one round of test" refers to a unit of continuous acquisition of pulse wave information by the pulse wave measuring device 100, and for example, the first period corresponding to one round of test is approximately several to ten days. However, since the battery or the like of the pulse wave measuring device 100 has its constraints, there may be a blank period during which no pulse wave information is acquired, during one round of test. The blank period is approximately several minutes to several hours, which is sufficiently short in relation to the first period. A time span of several days to several months is not considered. On the other hand, in the case where a plurality of tests is conducted, there is a blank period of approximately several days (if the efficiency of the test, the burden on the patient and the like are considered, several weeks) to several months between the individual tests.

Thus, when the first information is displayed, it is possible to present more detailed information as shown in FIG. 8A. In FIG. 8A, a pulse wave abnormality period can be identified on the basis of the presence/absence of a frame indicated by dashed lines and its position on the horizontal axis. Meanwhile, when the second information is displayed, though it is not easy to display detailed information, the outline of the state of the patient such as changes in time series in the result of test can be grasped easily. In FIG. 8B, a pulse wave abnormality period can be identified on the basis of the presence/absence of a vertical line and its position. The second information is, in other words, information indicating occurrence record information of atrial fibrillation. That is, proper information can be presented according to the granularity of information that the viewer wants. FIG. 8B shows a screen where the results of five rounds of tests are displayed together. FIG. 8A shows a screen where details of the result of the fifth round, of these tests, are displayed.

The action information, in a narrow sense, is expected to be displayed on the screen of FIG. 8A. In FIG. 8A, pulse wave abnormality periods are identifiably displayed via display objects (dashed-line frames), and the action information corresponding to FIG. 3A is displayed, as in A7 of FIG. 2. In FIG. 8A, LF and HF, which are autonomic nerve activity information, and AF rate, are displayed.

LF and HF are found by frequency conversion of time-series data of the pulse interval. Of the frequency-converted data, a relatively low frequency peak corresponds to LF, and a relatively high frequency peak corresponds to HF. Specifically, the powers of the respective peaks may be used as the values of LF and HF. LF shows a slow change in the pulse interval and mainly reflects the activity of sympathetic nerves. Meanwhile, HF shows a quicker change in the pulse interval than LF and mainly reflects the activity of parasympathetic nerves. In the example of FIG. 8A, the two values of HF and LF/HF are found and each value is associated with the result of analysis corresponding to the pulse wave abnormality periods in time series. Specifically, sharing the horizontal axis (time), the values of HF and LF/HF are plotted in the chart. If the value of HF is large, it can be determined that parasympathetic nerves are dominant, whereas if the value of LF/HF is large, it can be determined that sympathetic nerves are dominant.

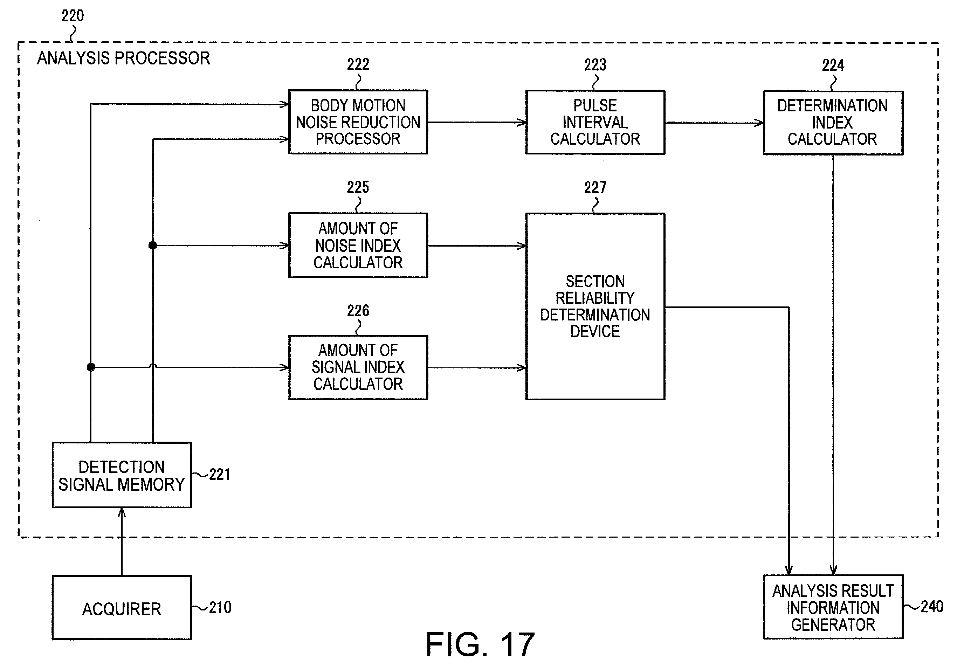

The AF rate is one of reliability indexes. As described later, a pulse wave abnormality period can be detected on the basis of whether the power in a certain frequency band, of temporal changes in the pulse interval, is above a predetermined threshold Pth or not. The AF rate in this example is information indicating the period of time during which the power is above Pth, in the processing target period. A greater value of the AF rate (closer to 100%) indicates higher reliability. Here, the processing target period is determined as a pulse wave abnormality period if the AF rate is above a predetermined threshold (for example, 50%) . That is, in this case, both of the determination index for the pulse wave abnormality period and the reliability index are the AF rate, and therefore this is a modification example which is different from the method in which the determination index and the reliability index are found by separate processes as shown in FIG. 17, described later.

Here, the analysis result information for identifiably displaying the pulse wave abnormality period can be considered to be information including continuous occurrence duration information of atrial fibrillation. The duration of continuous occurrence of atrial fibrillation relates to the likelihood of formation of a thrombus and the seriousness of symptoms caused by the thrombus. Therefore, by finding the continuous occurrence duration information indicating the duration of continuous occurrence of atrial fibrillation by analysis processing, and including this information in the analysis result information, it is possible to properly present important information to the viewer. Particularly, if the pulse wave abnormality period is displayed using a display object as shown in FIG. 8A, the correspondence between the duration of continuous occurrence and time and date can be easily achieved.

The continuous occurrence duration information may be any information indicating the duration of continuous occurrence, and specifically, it may be information such as x minutes or y hours. It is known that if the duration of continuous occurrence is approximately several minutes to one hour, a thrombus is less likely to be formed, whereas if the duration is approximately five hours, a thrombus is more likely to be formed. Therefore, of the duration of continuous occurrence acquired from the result of one or a plurality of rounds of tests, information indicating the maximum duration may be used as continuous occurrence duration information. The continuous occurrence duration information in this case may be text information such as "duration of continuous occurrence: three hours", or may be expressed by various graphs.

In the example of FIG. 8B, the horizontal axis represents date of test, and the vertical axis represents time of the day. In this case, one day (24 hours) corresponds to one vertical line. On the display screen as a whole, pulse wave abnormality periods over the entire test period can be expressed by lines corresponding to the number of days of test. In the example of FIG. 8B, no line is drawn for periods that are not pulse wave abnormality periods, and lines are drawn corresponding to the pulse wave abnormality periods.

As shown in FIG. 8B, information indicating the total duration of occurrence found by summing up the durations of occurrence of atrial fibrillation may be used. Specifically, the cumulative duration of pulse wave abnormality periods may be used as the total occurrence duration information. The line graph indicated by D1 in FIG. 8B shows changes in time series in the total occurrence duration information. The total occurrence duration information in this example is found by cumulative processing including the past tests. Each of points on D1 indicates the total duration of occurrence. Thus, changes in the condition of the disease can be easily understood on the basis of the slope of the line graph. In the example of FIG. 8B, the condition of the patient is not very good in the first to third tests, where relatively large slopes continue. However, in the fourth and fifth tests, the slope is gentler and it can be seen that the condition of the disease is improved.

The analysis result information may also display various kinds of information, as well as the result of determination on the pulse wave abnormality period and the action information or the like. For example, the processor 230 may generate the analysis result information including information associated with prescription drug record information indicating drugs prescribed to the user. In the example of FIG. 8B, the type (or name) of the drug prescribed after each of the first to fifth tests is displayed in the part (D2) above the frame showing the tests. In the example of FIG. 8B, warfarin is prescribed after the first and second tests, and beta blocker is prescribed in addition to warfarin after the third to fifth tests.

Thus, it is possible to confirm the record of prescribed drugs on the test result screen, and to easily determine whether the prescribed drugs are having effects (whether the patient is recovering from the disease, and the pulse wave abnormality period is becoming shorter, or the like) . For instance, in the example of FIG. 8B, since no improvement is observed in the first to third tests, it can be determined that the prescription of warfarin alone is not sufficiently effective. As beta blocker is prescribed after the third test, an improvement is observed in the subsequent fourth and fifth tests. Therefore, it can be determined that beta block is effective.

There are also cases of atrial fibrillation where the patient has no subjective symptoms. In the case of asymptomatic atrial fibrillation, the condition of the disease may get worse while the patient is unaware of the disease. Therefore, not only the presence/absence of occurrence but also information of whether the patient is aware of the occurrence or not is important. Thus, the processor 230 may generate the analysis result information including information in which event information indicating that the pulse wave measuring device 100 is operated is associated with a pulse wave abnormality period.

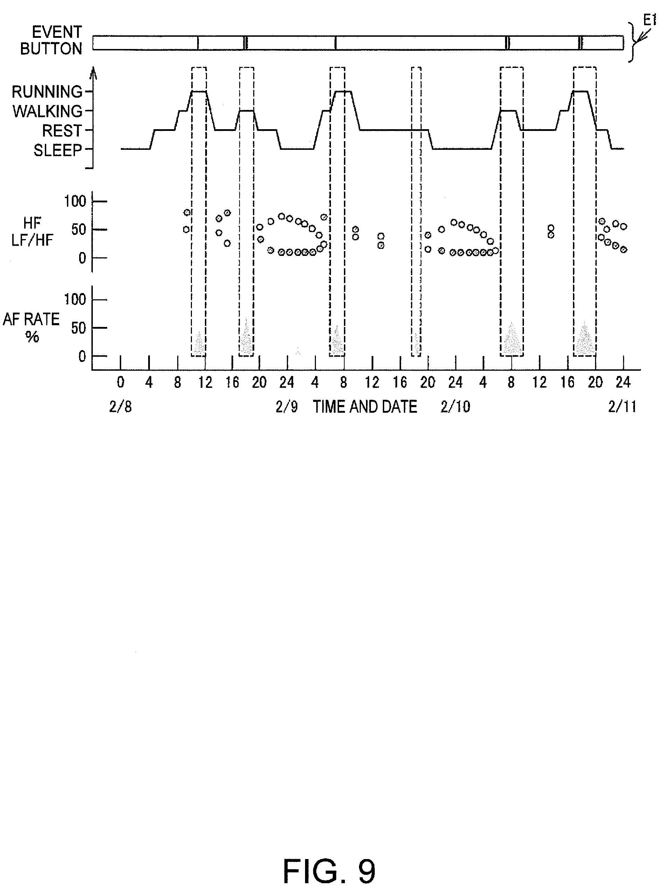

A display example of the analysis result information in this case is shown in FIG. 9. An item "event button" is provided in the part indicated by E1. Also in this part, the horizontal axis is time, as in the other parts, and a vertical line is displayed at the position of the timing when an operation is carried out by the user (the event button is pressed). In the example of FIG. 9, it can be seen that there are no subjective symptoms during the one pulse wave abnormality period around 18 : 00 on February 9, of the six pulse wave abnormality periods during tests, and that there are some subjective symptoms during the other five pulse wave abnormality periods.

Also, a display to clarify the effects of a procedure that is already carried out may be shown. The procedure may be, for example, catheter ablation. Catheter ablation is a treatment in which a catheter is inserted into the heart and then electrified so as to cauterize a part of the heat (for example, the site that causes tachycardia).

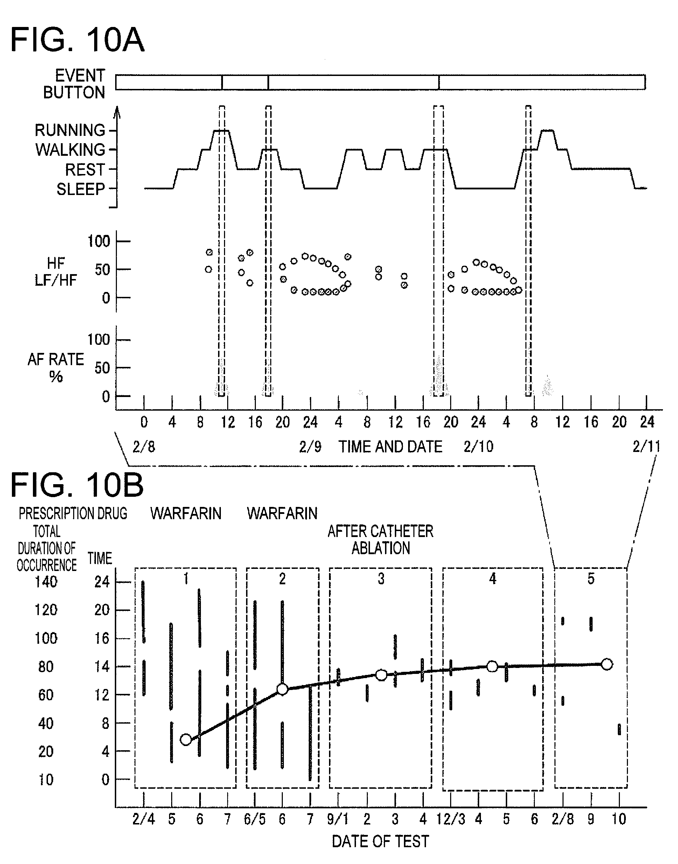

A display example of the analysis result information in this case is shown in FIGS. 10A and 10B. It is conceivable that the viewer wants to confirm effects on the basis whether the symptoms change around the catheter ablation or not. Therefore, the occurrence record information shown in FIG. 10B may be displayed. In the example of FIG. 10B, it can be seen that catheter ablation is carried out between the second and third tests and that the condition of the disease is improved by the catheter ablation. In the sense of confirming the effects, information indicating the timing when catheter ablation is carried out may be associated with the occurrence record information, as shown in FIG. 10B.

2.4 Modification 3 (Second Analysis Result Information)

Using the analysis result information as described above, it is possible to identify a pulse wave abnormality period during a measurement period and therefore allow the doctor to easily grasp the outline of the condition of an arrhythmia during the measurement period. That is, even if the measurement period is such a long period as three to ten days, at what timing or at what frequency an arrhythmia is suspected can be easily grasped, and information for specifying a factor or the like in the arrhythmia can be viewed as well. Therefore, the burden on the doctor can be reduced.

However, an arrhythmia should be diagnosed by the doctor and it is not desirable to present only the result of the determination by the biological information processing system 200. In other words, the output (analysis report or the like) from the biological information processing system 200 must also present information that is detailed enough for the doctor to diagnose whether there is an arrhythmia or not. Particularly, the pulse wave information is influenced by the body motion noise or the like and has lower accuracy than in the case of using an electrocardiogram. Therefore, there is a great need for a screen display or the like for checking whether the result of detection of a pulse wave abnormality by the system is appropriate or not.

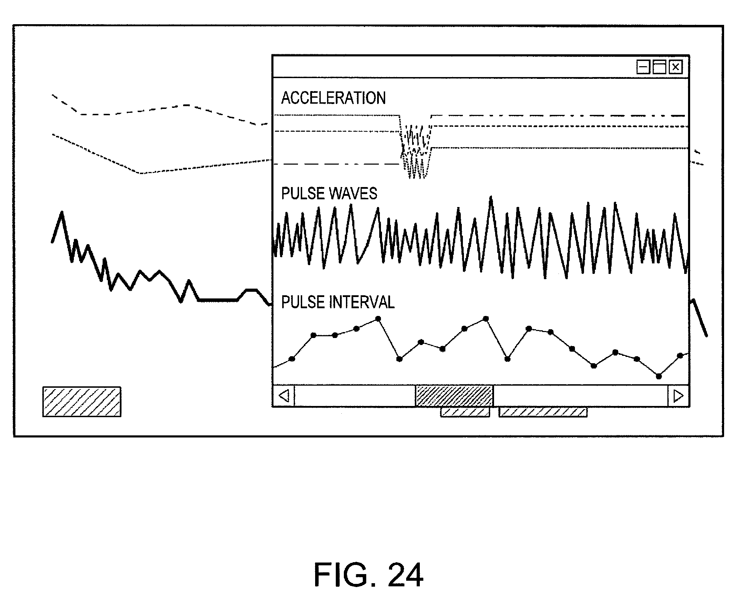

The analysis result information described above (in a narrow sense, the first analysis result information) displays information over a long period of four hours in FIG. 2 and 24 hours in FIG. 7. Therefore, this information is not suitable for the display of information with small changes in time series, such as the pulse wave information acquired at a rate of 16 Hz and the pulse interval (pulse rate) acquired once every second. For example, the display of the pulse interval in FIG. 2 is for the purpose of grasping the trend of the distribution (dispersion) of dots, rather than paying attention to each dot. In the display of the pulse rate in FIG. 7, the information has to be thinned to a certain extent. Also, in FIGS. 2 and 7, it is difficult to display a pulse waveform acquired at a higher rate.

That is, only with the display of the first analysis result information, the displayed information is insufficient with some information missing, compared with the pulse wave information and the body motion information acquired by the acquirer 210. It is inappropriate for the doctor to make a diagnosis of an arrhythmia, solely based on such insufficient information. For the doctor to make a diagnosis, a more detailed display is needed, such as displaying the pulse wave information itself or displaying each value of the pulse rate and the pulse wave information in a sufficiently identifiable form, for example.

In the embodiment, since the result of the determination on whether it is a pulse wave abnormality period or not is acquired in the first analysis result information, as described above, representative information during the pulse wave abnormality period may be used as more detailed information. Thus, information with relatively high importance in diagnosing an arrhythmia can be viewed in detail.

Thus, in addition to the analysis result information described above, second analysis result information for displaying more detailed information than the analysis result information may be generated.

For example, as shown in FIG. 11, the second analysis result information may be information which displays changes with time in the body motion information (acceleration information), the pulse waveform and the pulse interval waveform over a relatively short period of approximately 20 seconds. As shown in FIG. 11, as the display target section is limited to approximately 20 seconds, changes in the acceleration waveform, the pulse waveform and the pulse interval waveform can be displayed in detail (without thinning or the like). The section within the pulse wave abnormality period displayed via the second analysis result information is not limited to 20 seconds and may be 60 seconds or the like. Various modifications are possible.

As shown in the example of displaying the acceleration waveform in FIG. 11, the processor 230 may generate information for displaying at least one of the body motion information and the wearing state information during a predetermined section in the pulse wave abnormality period, as the second analysis result information. In the analysis result information described above, it is assumed that section reliability is used as the reliability index. The section reliability is determined, for example, with respect to a predetermined 20-second section or the like. Details of the section reliability will be described later in the explanation of a section reliability determination device 227.

That is, in the analysis result information (in a narrow sense, the first analysis result information), it is assumed that the reliability index of pulse wave measurement is displayed on a 20-second basis, and displaying details of the body motion information and the wearing state information used when finding the reliability index (for example, changes in time series during the 20 seconds) or the like is not particularly considered. However, with respect to the determination on whether the pulse wave measurement during the target section is reliable or not, it is desirable that the viewer (doctor) checks the result of the determination by the biological information processing system 200, instead of directly using the result of the determination.

That is, in the second analysis result information, though displaying the result of the determination by the section reliability determination device 227 is not precluded, more detailed information used for reliability determination may be displayed. Specifically, the waveform of the body motion information itself or the waveform of the wearing state index value itself may be displayed. For the determination of the wearing state, the signal level of the pulse wave information may be used. Therefore, the pulse waveform shown in FIG. 11 and the like is information that can have both a first aspect as detailed information for diagnosing the patient's arrhythmia and a second aspect as detailed information for determining reliability.

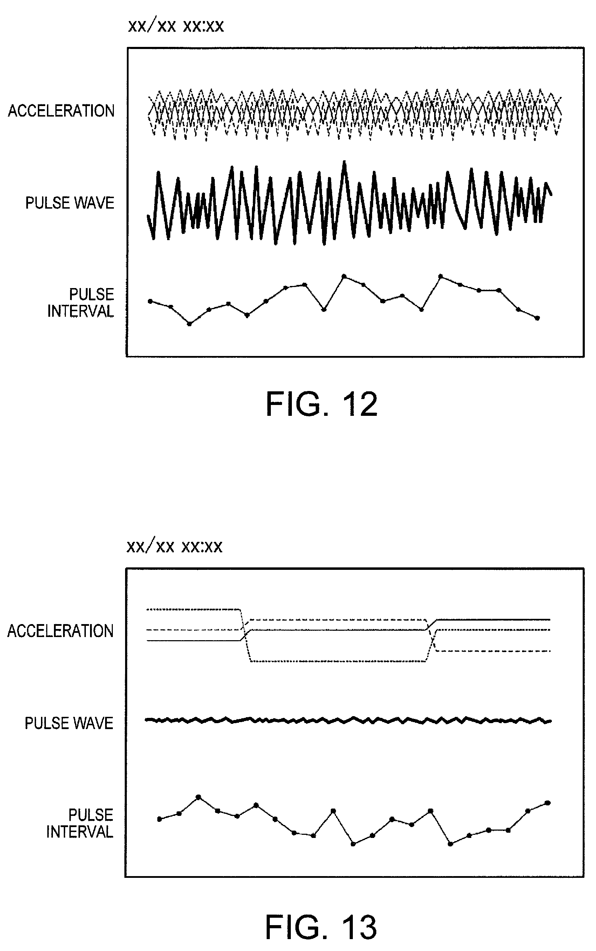

Other examples of the second analysis result information are shown in FIGS. 12 and 13. In FIG. 12, from the acceleration waveform displayed via the second analysis result information, it can be understood that the variation in value is large. Therefore, the influence of the body motion noise can be suspected. In FIG. 13, from the wearing state information (pulse waveform) displayed via the second analysis result information, it can be understood that the signal level is very low. Therefore, the influence of wearing failure can be suspected.

Thus, by using the second analysis result information, it is possible to display detailed information such as the pulse waveform or the pulse interval waveform corresponding to a predetermined section in the pulse wave abnormality period. Therefore, it is possible to allow the doctor to diagnose whether an arrhythmia truly occurs during the pulse wave abnormality period or not, using this detailed information.

Since the arrhythmia can occur a plurality of times (a plurality of episodes of arrhythmia can occur) during a period of three to ten days, the pulse wave abnormality period, too, can be detected a plurality of times during the measurement period, as shown in the first analysis result information of FIG. 7. In this case, in order for the doctor to make a proper diagnosis with respect to each pulse wave abnormality period, at least one section may be set in each pulse wave abnormality period and the display via the second analysis result information may be carried out, targeting this section.

That is, the processor 230 generates second analysis result information that can display at least one episode waveform with respect to one pulse wave abnormality period in which an episode of arrhythmia (continuous occurrence of arrhythmia) is suspected. The episode waveform in this example is information showing a representative waveform during a pulse wave abnormality period in which an episode of arrhythmia is suspected. The episode waveform corresponds to each of FIGS. 11 to 13. Consequently, the second analysis result information is information for displaying a plurality of episode waveforms.

In this case, the plurality of episode waveforms may be displayed, arranged in time series order, and the doctor may view the episode waveforms in the order of detection of the pulse wave abnormality periods. However, in the embodiment, the calculation of the reliability index is carried out. Therefore, with respect to each of the plurality of episode waveforms, how high the reliability of the pulse wave measurement is can be estimated.

With an episode waveform with high reliability, the detection accuracy of the pulse wave abnormality period is expected to be high. Therefore, there is a high possibility that the pulse wave abnormality period is detected because of an abnormality in the patient. For the doctor, viewing this episode waveform is useful for diagnosing an arrhythmia. Meanwhile, with an episode waveform with low reliability, there is a possibility that the pulse wave abnormality period is detected because of a noise even though the patient has no abnormality (false positive). Therefore, viewing this episode waveform might be useless for diagnosing an arrhythmia.

If the second analysis result information includes a plurality of episode waveforms, the degree of priority in display may be differentiated among these episode waveforms according to their reliability, instead of handling them equally. Specifically, if the reliability index for a first pulse wave abnormality period is higher than the reliability index for a second pulse wave abnormality period, the processor 230 generates second analysis result information in such a way that the analysis result information corresponding to the first pulse wave abnormality period is preferentially displayed over the analysis result information corresponding to the second pulse wave abnormality period.

Thus, the analysis result information (episode waveform) corresponding to the first pulse wave abnormality period, which has higher reliability and in which it is suspected that the patient truly has an arrhythmia, is displayed preferentially. Therefore, the doctor can preferentially view the useful information for diagnosing the arrhythmia. In contrast, the analysis result information (episode waveform) corresponding to the second pulse wave abnormality period, which has lower reliability and in which a false positive is suspected, has a lower degree of priority. Therefore, the doctor can leave until later the viewing of the information that can be useless for diagnosing the arrhythmia, or the like.

In the description below, an analysis report will be used as an example of the output of the biological information processing system 200 including the first and second analysis result information. Although the timing of preparation, the frequency of preparation and the like of the analysis report can be implemented with various modifications, in this example, a report showing a result corresponding to one round of test, that is, a report prepared by storing data over a period of about three to ten days, will be described. In this case, "differentiating the degree of priority among a plurality of episode waveforms included in the second analysis result information" can be achieved by differentiating the display form of the episode waveform in the analysis report.

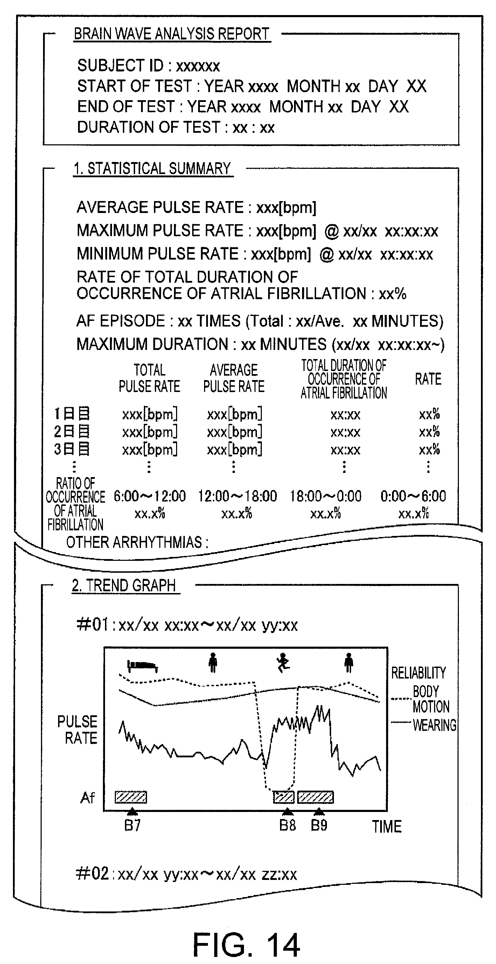

FIGS. 14 and 15 show an example of the analysis report in the embodiment. The analysis report is made up of four items, that is, statistical summary, trend graph, episode waveform, and full waveform.

The statistical summary is an item showing a representative statistical quantity indicating the state of the user (patient) during the testing period. For example, an average value, maximum value, minimum value and the like of pulse rate are displayed, as shown in FIG. 14. The maximum pulse rate and the minimum pulse rate may be displayed along with the timings (month, day, hour, minute, second) when these pulse rates are detected, as shown in FIG. 14. Also, the number of times it is determined that atrial fibrillation (Af) has occurred, total duration, average duration per occurrence, maximum duration and the like are displayed. The maximum duration may be displayed along with the timing of occurrence (occurrence start timing, end timing) of this atrial fibrillation.

Also, the display processor may perform display processing in which at least one of the total pulse rate per day, the average pulse rate per day, the rate of the total duration of occurrence of atrial fibrillation to the duration of test, the total duration of occurrence of atrial fibrillation per day, the rate of the total duration of occurrence of atrial fibrillation per day to the duration of test, and the rate of occurrence of atrial fibrillation in a designated time bracket, as shown in FIG. 14.

Here, if the duration of test is 120 hours (five days), and the total duration of occurrence of atrial fibrillation per day is one hour for the first day, two hours for the second day, 0 hours for the third day, three hours for the fourth day, and four hours for the fifth day, the rate of the total duration of occurrence of atrial fibrillation to the duration of test is 0.83% for the first day, 1.67% for the second day, 0.00% for the third day, 2.50% for the fourth day, and 3.33% for the fifth day.

The rate of occurrence of atrial fibrillation in designated time bracket may be a value calculated by totaling respective designated time brackets of all test days, or the rate of occurrence of atrial fibrillation in a designated time bracket of each day may be calculated, as shown in FIG. 14.

Thus, it is possible to display information useful for the doctor to determine the frequency of occurrence of atrial fibrillation or whether the occurrence of atrial fibrillation concentrates on a specific day or not, or the like. This is because, if the occurrence of atrial fibrillation concentrates on a specific day, the symptoms are likely to have become worse. Also, as these types of information, the corresponding information of the day may be displayed on the second screen where the pulse waveform or the like per day is displayed.

The full waveform shows the acceleration waveform, the pulse waveform, the pulse interval waveform and the state of occurrence of atrial fibrillation (result of determination of pulse wave abnormality period) over the entire testing period, as shown in FIG. 15.

As can be understood from FIG. 14, the statistical summary is suitable for grasping the summary of the testing period in the form of numerical values, but is unsuitable for grasping changes in time series in the state of the user because no specific waveform or the like is displayed. Therefore, more detailed information is necessary for the doctor to make a diagnosis. Also, as can be understood from FIG. 15, the full waveform displays detailed information but results in an excessively large amount of information. Therefore, properly summarized information or important information extracted from the whole needs to be displayed for the doctor to make a diagnosis.

As information that facilitates the diagnosis by the doctor, the first analysis result information and the second analysis result information are generated in the embodiment, as described above. The first analysis result information in the embodiment corresponds to the trend graph in FIG. 14, for example. The second analysis result information corresponds to the episode waveform in FIG. 15, for example. The episode waveform in this example is information indicating a representative waveform during a pulse wave abnormality period suspected of having an arrhythmia episode and corresponds to each of FIGS. 11 to 13.

As can be seen from the trend graph of FIG. 14 (FIG. 7), a plurality of pulse wave abnormality periods may be detected in the measurement period. Consequently, the second analysis result information is information for displaying a plurality of episode waveforms.

In this case, the plurality of episode waveforms may be displayed, arranged in time series order, and the doctor may view the episode waveforms in the order of detection of the pulse wave abnormality periods. However, in the embodiment, the calculation of the reliability index is carried out. Therefore, with respect to each of the plurality of episode waveforms, how high the reliability of the pulse wave measurement is can be estimated.

With an episode waveform with high reliability, the detection accuracy of the pulse wave abnormality period is expected to be high. Therefore, there is a high possibility that the pulse wave abnormality period is detected because of an abnormality in the patient. For the doctor, viewing this episode waveform is useful for diagnosing an arrhythmia. Meanwhile, with an episode waveform with low reliability, there is a possibility that the pulse wave abnormality period is detected because of a noise even though the patient has no abnormality (false positive). Therefore, viewing this episode waveform might be useless for diagnosing an arrhythmia.

If the second analysis result information includes a plurality of episode waveforms, the degree of priority in display may be differentiated among these episode waveforms according to their reliability, instead of handling them equally. Specifically, if the reliability index for a first pulse wave abnormality period is higher than the reliability index for a second pulse wave abnormality period, the processor 230 generates second analysis result information in such a way that the analysis result information corresponding to the first pulse wave abnormality period is preferentially displayed over the analysis result information corresponding to the second pulse wave abnormality period.

Thus, the analysis result information (episode waveform) corresponding to the first pulse wave abnormality period, which has higher reliability and in which it is suspected that the patient truly has an arrhythmia, is displayed preferentially. Therefore, the doctor can preferentially view the useful information for diagnosing the arrhythmia. In contrast, the analysis result information (episode waveform) corresponding to the second pulse wave abnormality period, which has lower reliability and in which a false positive is suspected, has a lower degree of priority. Therefore, the doctor can leave until later the viewing of the information that can be useless for diagnosing the arrhythmia, or the like.

Specific examples of the technique for preferentially displaying the analysis result information (episode waveform) corresponding to a predetermined pulse wave abnormality period over other analysis result information are conceivable. For example, the processor 230 may generate the second analysis result information in such a way that the analysis result information corresponding to the first pulse wave abnormality period is displayed earlier than the analysis result information corresponding to the second pulse wave abnormality period.

In the case of outputting a report on a paper medium, pdf file or the like as the output including the first and second analysis result information (for example, analysis report), this analysis report shows a plurality of items in a fixed order, and no change or the like is made to the order. An item which comes earlier in the order of display is displayed at a position closer to the beginning (first page), whereas an item which comes later in the order of display is displayed at a position closer to the end (last page). The page order in which the analysis report is viewed varies depending on the viewer. However, it is generally expected that viewing starts with an item close to the first page. That is, "being displayed earlier" in this example may be being arranged at a position closer to the first page.

The trend graph of FIG. 14 corresponds to FIG. 7. In this example, as can be understood from the graph of the reliability index, the reliability corresponding to a section B7 is high, whereas the reliability corresponding to a section B8 is low. In this case, the episode waveform corresponding to B7 (corresponding to FIG. 11) is displayed as in C1 in FIG. 15, and the episode waveform corresponding to B8 (corresponding to FIG. 12) is displayed as in C2 in FIG. 15. That is, the episode waveform corresponding to B7 with high reliability is displayed at a position closer to the first page. Also, the episode waveform corresponding to FIG. 13 has low reliability and therefore may be displayed more to the back (subordinately) as in C3 in FIG. 15.

Alternatively, each kind of information that is a display target may be associated with information indicating the display order. For example, the analysis result information corresponding to the first pulse wave abnormality period may be provided with first internal data (first metadata) indicating the display order, and the analysis result information corresponding to the second pulse wave abnormality period may be provided with second internal data (second metadata) indicating the display order. The data indicating the display order is provided in a format that enables comparison between data (for example, numerical data that can be determined as large or small). Thus, by comparing the first internal data with the second internal data, which comes earlier in the order can be determined. For example, if the internal data is numerical data and the position in the order of display becomes earlier as the numerical value becomes smaller, two internal data are generated in such a way that the first internal data < the second internal data, and each of these is provided for the analysis result information corresponding to each pulse wave abnormality period. This enables differentiation in the degree of priority. In the case of deciding the order of display using such internal data, processing of generating display images that are put in order on the basis of the internal data may be performed by the processor 230 according to the embodiment or may be performed by a processor in a device having a display. Also, the internal data may be provided for other types of information such as the first analysis result information as well.

It is conceivable that the viewer follows the procedure of grasping the summary over the entire measurement period from the first analysis result information and then making a detailed diagnosis using the second analysis result information. That is, when a display area for displaying the second analysis result information is set, the degree of priority of the viewing by the viewer is considered to be higher as the display position (arrangement position) in the display area is closer to the first analysis result information. That is, "being displayed earlier" may be being arranged at a position closer to the first analysis result information.

Also, "being preferentially displayed" may be based on whether the display area (display size) is large or small. The second analysis result information may be generated in such a way that the analysis result information corresponding to the second pulse wave abnormality period is displayed in a reduced size, compared with the analysis result information corresponding to the first pulse wave abnormality period. Thus, since the episode waveform with high reliability is displayed in a relatively large size and the episode waveform with low reliability is displayed in a relatively small size, the user can easily understand which episode waveform the user should pay attention to.

In the example of FIG. 15, the episode waveform with low reliability (C2 and C3 in FIG. 15, corresponding to FIGS. 12 and 13) is reduced in size to 1/4 of the episode waveform with high reliability (C1 in FIG. 15, corresponding to FIG. 11), and the visibility of the episode waveform with high reliability is relatively high.

The method for "preferentially displaying" is not limited to whether the position in the order is earlier or later, or whether the display size is large or small, and may be implemented by various kinds of highlighting processing such as changing the background color or changing the color or size of letters.

Up to this point, the description is based on the case where detailed information corresponding to a predetermined section in a pulse wave abnormality period, that is, only an episode waveform, is displayed. However, this example is not limiting. For instance, in some cases, depending on the objective of diagnosis or the person making a diagnosis, it is demanded that a space for showing a representative non-episode waveform (detailed information corresponding to a section that is not a pulse wave abnormality period) should be added to the analysis report.

In such a case, the analysis result information for displaying the body motion waveform, the pulse waveform and the pulse interval waveform may be generated, for example, similarly to FIG. 11 or the like, targeting a section that is not a pulse wave abnormality period. In this case, instead of handling the respective non-episode waveforms equally, the degree of priority corresponding to the reliability may be set, or a non-episode waveform with high reliability may be displayed earlier or displayed in a relative large size.

Also, while it is assumed in the above description that the output including the first and second analysis result information is in a static format, the output according to the embodiment may be in a dynamic (interactive) format. For example, a pulse wave abnormality period may be designated on the first analysis result information (trend graph), so that a representative waveform (second analysis result information, episode waveform) corresponding to the designated pulse wave abnormality period will be displayed. The display of the representative waveform in this example may be opening a new display window or may be jumping to a display site. Other formats may also be employed. In this case, since the concept of whether the position in the order of display is earlier or later is insubstantial, highlighting such as changing the background color may be carried out as the method for "preferentially displaying".

An example of display of the first and second analysis result information in a dynamic format is shown in FIG. 24. FIG. 24 shows an example where a representative waveform is displayed by opening a new display window. In FIG. 24, it is assumed that the pulse wave abnormality period corresponding to B4 (B7) in FIG. 7 is designated in the state where the trend graph shown in FIG. 7 is displayed. A new display window (popup screen) is superimposed on the trend graph of FIG. 7. In the new display window, the representative waveform corresponding to FIG. 11 is displayed. Also, the display of the representative waveform is not limited to the fixed display of a waveform corresponding to a section with a predetermined length (for example, 20 seconds), and the transition of the waveform with time before and after the section may be displayed. For example, in FIG. 24, a scroll bar is provided at the bottom of the new display window, and the user can change the section to be the waveform display target by operating the scroll bar. For example, the user can cause the waveform that is earlier in time series to be displayed by pressing the left arrow button in the scroll bar in FIG. 24 or moving the scroll box to the left. The user can cause the waveform that is later in time series to be displayed by pressing the right arrow button or moving the scroll box to the right. The user interface in the case of viewing the transition of pulse waves with time before and after the displayed representative waveform is not limited to the scroll bar and can be implemented with various modifications.

As described above, the display processor performs display processing in which a pulse wave abnormality period for determination on an arrhythmia is identifiably displayed on the first screen as the analysis result information, and performs display processing in which pulse interval information is displayed on the second screen as the original information. Then, the display processor performs display processing in which the pulse interval information corresponding to the pulse wave abnormality period is displayed on the second screen, when a predetermined instruction operation is carried out by the user on the display object indicating the pulse wave abnormality period displayed on the first screen.

For example, the first screen where the pulse wave abnormality periods are identifiable is displayed, as shown in FIG. 7, and when the viewer carries out a tap operation on image data of one of the pulse wave abnormality periods indicates by the bars B4 to B6, the second screen as shown in FIG. 11 is displayed. In the example of FIG. 7, the bars B4 to B6 are display objects indicating pulse wave abnormality periods. However, the display objects in the embodiment are not limited these.

Thus, it is possible to display the pulse interval information corresponding to the pulse wave abnormality period which the viewer wants to view, or the like.

Here, the predetermined instruction operation is at least one of a click operation, a touch operation, a tap operation, a pinch-in operation, a pinch-out operation, a scroll operation, and a swipe operation on the first screen. For example, if the operation device of the display device is a mouse or the like, the predetermined instruction operation is a click operation, a scroll operation or the like. If the display device has a touch panel-type display, the predetermined instruction operation is a touch operation, a tap operation, a pinch-in operation, a pinch-out operation, and a swipe operation or the like.

Thus, the viewer can easily designate a pulse wave abnormality period for which the viewer wants to view the pulse interval information, or the like.

When the predetermined instruction operation is carried out, the pulse interval information need not necessarily be displayed with respect to all the sections in the pulse wave abnormality period. For example, as shown in FIG. 24, only the pulse interval information corresponding to a section in the pulse wave abnormality period may be displayed. That is, the display processor 250 performs display processing in which the pulse interval information corresponding to a predetermined section in the pulse wave abnormality period is displayed on the second screen, when the predetermined instruction operation by the user is carried out on the display object indicating the pulse wave abnormality period displayed on the first screen.

Thus, it is possible to display the pulse interval information corresponding to the section which the user wants to view, of the pulse wave abnormality period, or the like.

Also, the user need not necessarily designate the predetermined section and the processor 230 may decide the predetermined section on the basis of reliability of pulse wave measurement.

Thus, it is possible to preferentially display the pulse interval information corresponding to a period with high reliability, for example, without displaying the pulse interval information corresponding to a period with low reliability in pulse wave measurement, or the like.

Also, the processor 230 generates information for displaying the pulse wave abnormality period and a pulse wave normality period which is a period that is not the pulse wave abnormality period, in different image display forms from each other, as the analysis result information. The display processor 250 displays the bars B4 to B6 corresponding to the pulse wave abnormality periods, for example, on the first screen as shown in FIG. 7, and thus enable discrimination between the pulse wave abnormality periods and pulse wave normality periods. However, the image display form of the pulse wave abnormality periods in the embodiment is not limited to this example. For example, the color of the waveform may be changed between the pulse wave abnormality period and the pulse wave normality period, or one of the periods may be displayed in a hatched image.

Thus, it is possible for the viewer to easily discriminate between the pulse wave abnormality period and the pulse wave normality period, or the like.

Also, the display processor 250 performs display processing in which the analysis result information over the entire test period, or the analysis result information over a predetermined period of one day is displayed on the first screen.

For example, the analysis result information over the entire test period is useful for quickly grasping the overall trend during the test period, whereas the analysis result information over a predetermined period is useful for grasping changes in the trend in every predetermined period. The predetermined period is one day, for example. However, this is not limiting.

Thus, it is possible to display the analysis result information corresponding to the period which the viewer wants to view, on the first screen, or the like.

Also, during the test period, the subject himself/herself may feel a pulse wave abnormality. The information of the timing when the subject becomes aware of the pulse wave abnormality can be used as very effective information at the time of diagnosing an arrhythmia. Thus, in the embodiment, for example, an operation device such as a button is provided on the pulse wave measuring device worn by the subject, and the subject is asked to press the button or the like when becoming aware of a pulse wave abnormality. Then, the display processor 250 may display a mark at the position on the first screen corresponding to the timing when the operation by the user when becoming aware of the pulse wave abnormality is detected. For example, in FIG. 15, a mark US is displayed.

Thus, the doctor can determine whether it is an arrhythmia or not, after grasping the timing when the subject himself/herself feels a pulse wave abnormality.

3. Example of System Configuration

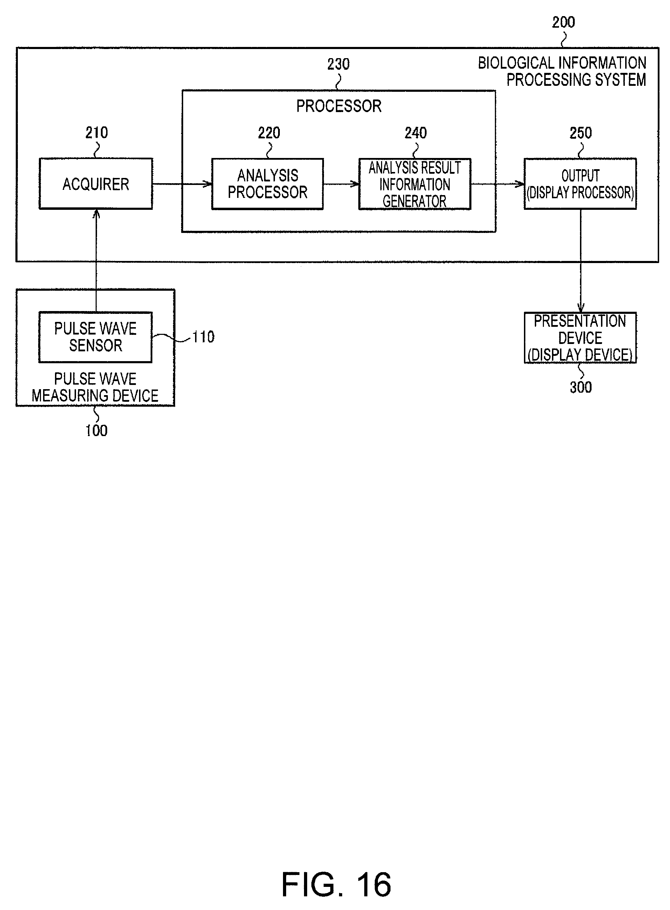

Next, an example of the configuration of the biological information processing system 200 or the like according to the embodiment will be described. First, a specific example of the system configuration of the biological information processing system 200 will be described with reference to FIG. 16 and the like. Subsequently, an example of the appearance of a pulse wave measuring device 100 and a specific example of a system including the biological information processing system 200 will be described with reference to FIGS. 21A to 23.

3.1 Example of Configuration of Biological Information Processing System

As shown in FIG. 1, the biological information processing system 200 according to the embodiment includes the acquirer 210, the processor 230, and the output (display processor) 250.

The acquirer 210 acquires pulse wave information. For example, in the case where the biological information processing system 200 according to the embodiment is implemented by a server system and the server system acquires pulse wave information from the pulse wave measuring device 100 (biological information detection device) worn by the user, as described later with reference to FIG. 23A, the acquirer 210 may be a communication device which communicates with the pulse wave measuring device via a network (a receiver which receives information from the pulse wave measuring device).

In this case, the acquirer 210 acquires sensor information from a pulse wave sensor included in the pulse wave measuring device. Here, the pulse wave sensor is a sensor for detecting a pulse wave signal and may be, for example, a photoelectric sensor including a light emitter and a light receiver. It is known that the pulse wave sensor can be implemented by various sensors such as a photoelectric sensor and other types of sensors (for example, ultrasonic sensor). These sensors can be broadly applied as the pulse wave sensor in the embodiment.

The processor 230 performs analysis processing on atrial fibrillation on the basis of the pulse wave information acquired by the acquirer 210 and generates analysis result information on the basis of the result of the analysis. The functions of the processor 230 can be implemented by hardware such as various processors (CPU or the like) or ASIC (gate array or the like), or by a program or the like.

The output 250 outputs the analysis result information generated by the processor 230. Various output forms from the output 250 are possible. For example, the analysis result information may be transmitted to another device via a network. In this case, the output 250 is implemented by a communication device. The network in this case can be implemented by a WAN (wide area network), LAN (local area network) or the like, and may be wired or wireless. Alternatively, the output 250 may print the analysis result information. In this case, the viewer views the printed analysis result information on a paper medium or the like.

The processing by the processor 230 of generating the analysis result information for displaying various kinds of information may be processing of generating display screen information or may be processing of generating data for printing. Information for screen display (for example, an HTML file or the like prescribing the arrangement relation between the respective kinds of information) may be used. Generating the information for screen display may be, for example, processing of generating a page for viewing (for example, a web page).

The output 250 may be a display processor which displays a display screen prescribed by the display screen information, on the display of a terminal device. For example, a device including the biological information processing system 200 according to the embodiment may have a display, and the display screen may be displayed on this display. Or, the display screen may be displayed on the display of a terminal device which is different from the device including the biological information processing system 200. Alternatively, the output 250 may transmit the data for printing to a printing device. Alternatively, the output 250 may be a transmitter (communication device) which transmits the information for screen display in response to a request from a terminal device which is different from the device including the biological information processing system 200. In this case, a specific screen display using the information for screen display may be carried out on the side of the terminal device which has receives this information for screen display. As an example, in the case where a viewing request for a web page using a web browser is sent from a terminal device to the biological information processing system 200, the processor 230 may send back information of the web page corresponding to the viewing request, and the web browser of the terminal device may interpret the information of this web page and display the information on the display. However, the transmission and reception of information between the terminal device and the biological information processing system 200 is not limited to the transmission and reception using the web browser. For example, a native application may be used.

FIG. 16 shows a detailed example of the configuration of the system including the biological information processing system 200. However, the biological information processing system 200 is not limited to the configuration of FIG. 16 and can be implemented with various modifications such as omitting a part of the components or adding another component. In the example of FIG. 16, the acquirer 210 of the biological information processing system 200 acquires pulse wave information from the pulse wave measuring device 100 including a pulse wave sensor 110.

The processor 230 includes an analysis processor 220 which performs analysis processing on an arrhythmia (atrial fibrillation), and an analysis result information generator 240 which generates analysis result information.

FIG. 17 shows a detailed example of the configuration of the analysis processor 220. As shown in FIG. 17, the analysis processor 220 includes a detection signal memory 221, a body motion noise reduction processor 222, a pulse interval calculator 223, a determination index calculator 224, an amount of noise calculator 225, an amount of signal calculator 226, and a section reliability determination device 227.

The detection signal memory 221 stores the information acquired by the acquirer 210. Here, it is assumed that the acquirer 210 acquires body motion information (in a narrow sense, acceleration information) indicating the body motion of the user, as well as the pulse wave information. The detection signal memory 221 stores the pulse wave information and the body motion information. For example, the body motion information is information from an acceleration sensor included in the pulse wave measuring device 100.

The body motion noise reduction processor 222 acquires a pulse wave signal and a body motion signal from the detection signal memory 221 and performs processing of reducing the body motion noise on the pulse wave signal. The body motion noise reduction processor 222 can be implemented, for example, by adaptive filter processing or the like. In the embodiment, as described later, since the reliability index is used, even if the body motion noise is not eliminated and consequently an arrhythmia is erroneously detected (a false positive is detected), it is possible to lower the degree of priority of display for the result of this detection. Therefore, the body motion noise reduction processor 222 in the embodiment is not an essential component and can be omitted.

The pulse interval calculator 223 finds the pulse interval on the basis of the pulse wave information. If the influence of the noise on the pulse waveform is small, the peak interval or the like in the pulse waveform can be used as the pulse interval. However, the pulse waveform is usually not such a smooth and regular waveform. Therefore, the pulse interval may be found by frequency analysis processing on pulse wave information corresponding to a certain period. As an example, the pulse interval calculator 223 slices out, at each sampling, a frame of the pulse wave information with the body motion noise component reduced by the body motion noise reduction processor 222, and calculates a frequency spectrum by short-time frequency analysis (STFT (short-time Fourier transform) analysis). Then, the pulse interval calculator 223 calculates a parameter equivalent to the pulse interval for each frame on the basis of the frequency spectrum that is calculated.

The frame in this example may be, for example, a period of about four seconds. In the embodiment, the frame of four seconds or the like for which the pulse interval is found is also referred to as a pulse interval calculation period. In the description below, the pulse interval calculation period is a period of four seconds and this period is shifted by one second each, so as to find the pulse interval once very second. However, the length of the pulse interval calculation period and the rate of finding the pulse interval can be implemented with various modifications. The pulse interval that is found is outputted to the determination index calculator 224.

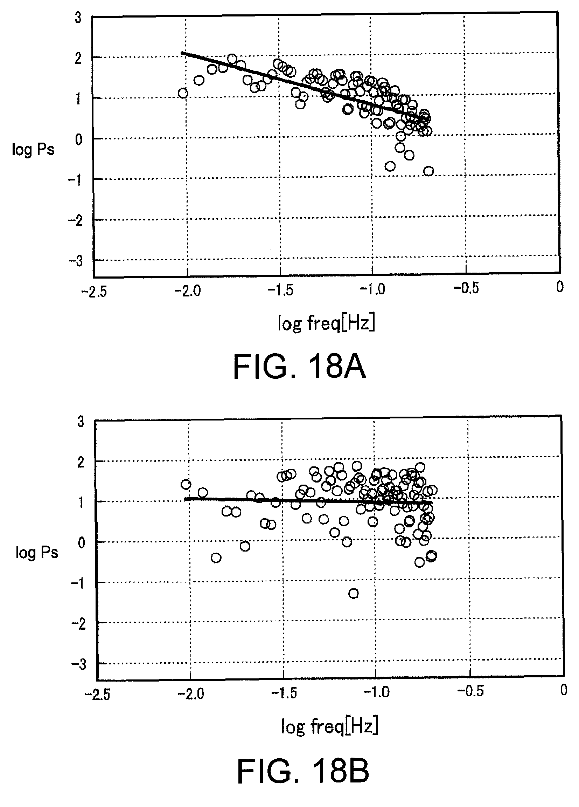

The determination index calculator 224 finds a determination index for determining whether an arrhythmia (atrial fibrillation) has occurred or not, specifically, whether it is a pulse wave abnormality period or not, on the basis of the pulse interval. FIGS. 18A and 18B are graphs showing the result of performing frequency analysis in a band from 0.01 Hz to 0.2 Hz is performed for one frame of a waveform signal indicating a variation in the electrocardiogram RR interval and then converting the peak frequency and power into logarithms. FIG. 18A is a graph in the case where atrial fibrillation has not occurred. FIG. 18B is a graph in the case where atrial fibrillation has occurred.

As can be understood from FIGS. 18A and 18B, when the electrocardiogram RR interval is used, the intercept and slope of the regression line changes according to whether atrial fibrillation has occurred or not. Therefore, it is possible to determine whether atrial fibrillation has occurred or not, from the intercept and slope. Thus, the determination index calculator 224 may find a graph showing the peak frequency and power converted into logarithms with respect to the pulse waveform showing the fluctuation in the pulse interval, and may find the intercept and slope of the graph as the determination index. By comparing the determination index with the intercept or the like in a normal state and the intercept or the like at the occurrence of atrial fibrillation, it is possible to determine whether it is a pulse wave abnormality period in which an abnormality is observed in the pulse wave information or not, specifically, whether atrial fibrillation has occurred or not. The determination index calculator 224 may output the result of determination based on the intercept or the like (result of determination on whether atrial fibrillation has occurred or not), instead of using the intercept and slope as index values.

Alternatively, the power in a part of the frequency band may be calculated, of the change with time in the pulse interval. When atrial fibrillation has occurred, the power increases by several times, showing a significant difference, compared with when atrial fibrillation has not occurred. Therefore, whether atrial fibrillation has occurred or not may be determined on the basis of the change in power.

It is known that the fluctuation in time series in the pulse interval increases at the occurrence of atrial fibrillation. Therefore, modifications such as finding a statistical quantity such as a variance value or standard deviation may be carried out. Also, in such a case, a statistical quantity of the amount of change (difference or proportion) from the pulse interval at the immediately preceding timing may be found, instead of the statistical quantity of the pulse interval itself. In this way, the processing by the determination index calculator 224 can be implemented with various modifications. In the description below, it is assumed that the result of determination on whether it is a pulse wave abnormality period or not is outputted from the determination index calculator 224.

The amount of noise calculator 225 finds an amount of noise index indicating the degree of noise included in the pulse wave information. Specifically, the amount of noise calculator 225 may acquire the body motion information (acceleration information) from the detection signal memory 221 and find an index value indicating the degree of the body motion noise. More specifically, as the amount of body motion noise, the power in a predetermined band of the amounts of accelerations on the three axes of the acceleration sensor can be used.

The amount of signal calculator 226 finds an amount of signal index indicating the amount of signal (signal level) included in the pulse wave information. The amount of signal calculator 226 may simply find the amplitude level (amount of effective signal or the like) of the pulse wave signal, as the amount of signal index. The analysis processor 220 is not limited to the configuration including both the amount of noise calculator 225 and the amount of signal calculator 226 and may be configured to include one of these calculators.

The processor 230 (in a narrow sense, the analysis processor 220 of the processor 230) also includes the section reliability determination device 227 which finds the reliability index corresponding to each section in the measurement period on the basis of at least one of the body motion information and the pulse wave information of the user.

The section reliability determination device 227 can find the reliability index based on the amount of noise index, for example, by a method such as extracting, with a digital filter, a predetermined band and finding an effective value from the acceleration waveform on each axis of the acceleration sensor, then multiplying the value by a coefficient corresponding to the degree of influence on pulse measurement with respect to each axis, and adding the results. The predetermined band in this example is, for example, 0.5 to 2 [Hz]. This corresponds to a band which can overlap with the frequency band of pulse. As the reliability index based on the amount of signal index, the amount of effective signal of the pulse wave signal may be used.

The length of each section in the measurement period to be the target of the section reliability determination in this example is, for example, approximately 20 seconds. In this case, the section reliability determination device 227 determines the reliability index (section reliability) corresponding to the period of 20 seconds on the basis of the amount of effective signal of the body motion information (acceleration signal) during the 20 seconds outputted from the amount of noise calculator 225 and the amount of effective signal of the pulse wave information during the 20 seconds outputted from the amount of signal calculator 226.

The analysis result information generator 240 of the processor 230 generates analysis result information on the basis of the determination index outputted from the determination index calculator 224 and the reliability index outputted from the section reliability determination device 227. Details of the analysis result information are as described above.

In the example of FIG. 16, the output 250 outputs the analysis result information to another presentation device 300. That is, FIG. 16 shows an example in which the analysis result information is transmitted via a network or the like, as described above. The presentation device 300 presents the analysis result information acquired from the output 250, to the viewer. In a narrow sense, the presentation device 300 may include a display and may display the analysis result information on the display.

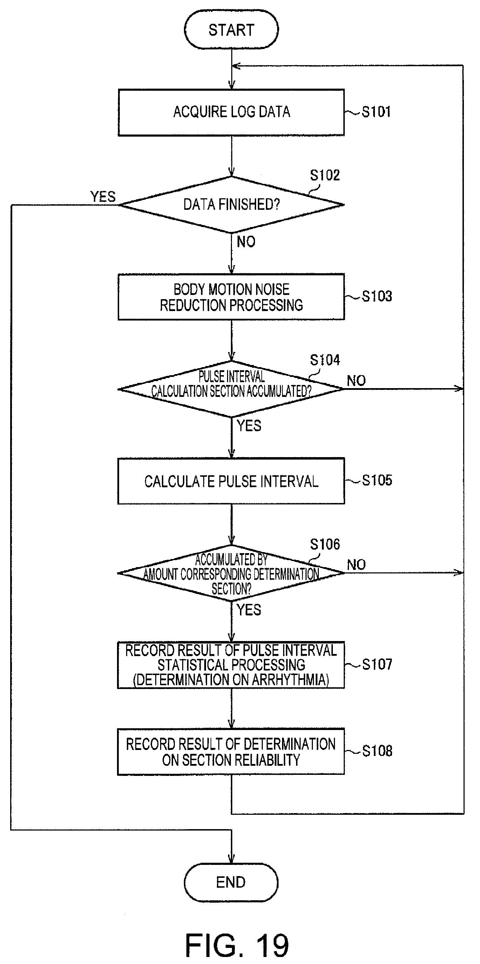

FIG. 19 shows a flowchart explaining the processing carried out by the analysis processor 220 in the biological information processing system 200 according to the embodiment. As this processing is started, first, log data acquired by the acquirer 210 is read (S101). The log data in this example is, for example, data of pulse wave information and body motion information accumulated over one entire measurement period of three to ten days. In S101, processing of reading one piece of data from among the entire data may be carried out.

Next, whether the processing on the log data is finished or not is determined (S102). For example, the processing may be performed sequentially from the beginning of the log data, and whether unprocessed data is left or not may be determined. If there is no unprocessed data (No in S102), the processing by the analysis processor 220 ends and the flow shifts to processing of generating analysis result information for display based on the result of the analysis.

If there is unprocessed data (Yes in S102), the processing of S103 and onward is performed. Specifically, first, the body motion noise reduction processor 222 performs processing of reducing the body motion noise (S103). The body motion noise reduction processing can be implemented, for example, by filter processing as described above.

Then, it is determined whether the pulse information after the reduction of the body motion noise is accumulated by the amount corresponding to the pulse interval calculation section or not (S104). Since the pulse interval calculation section is, for example, four seconds, the determination in S104 results in Yes when information corresponding to four seconds is accumulated. For example, if the sampling rate of the pulse wave information is 16 Hz, the information corresponding to four seconds is 64 pieces of data. If the result is No in S104, the flow returns to S101 to read the next log data. The pulse interval calculation section may be set with a shift of one second, as described above. In such a case, in the processing of S104, data corresponding to four seconds need not be accumulated from scratch, and already accumulated data may be utilized as data corresponding to three seconds. That is, in S104, the determination results in Yes once every second.

If it is Yes in S104, the pulse interval is calculated on the basis of the accumulated pulse wave information corresponding to the pulse interval calculation section (S105). The specific calculation method is as described above. The processing of S105 is executed at the same rate as the rate at which the determination results in Yes in S104. Therefore, the pulse interval is found, for example, one pulse interval per second.

Next, it is determined whether data is accumulated by the amount corresponding to a determination section for which reliability is determined (S106). The determination section in this example is, for example, 20 seconds. Therefore, in S106, the determination results in Yes once every 20 seconds, and otherwise it is No. In the case of No, the flow returns to S101 to read the next log data.

If it is Yes in S106, first, whether it is a pulse wave abnormality period or not is determined on the basis of the pulse intervals accumulated by the amount corresponding to the determination section (S107). Since the pulse interval is found, for example, once every second, the determination index calculator 224 may carry out this determination using changes in time series about 20 pulse intervals (21 pulse intervals as shown in FIG. 11 or the like, depending on the handling of the pulse intervals at the ends of the determination section).

The section reliability determination device 227 determines the section reliability on the basis of the pulse wave information and the body motion information corresponding to 20 seconds (S108). After the processing of S108, the flow returns to S101 to read the next log data.

By carrying out the processing shown in FIG. 19, the pulse interval can be found once every second, and the result of determination on the pulse wave abnormality period and the section reliability can be found once every 20 seconds. Also, since the pulse interval can be converted to find the pulse rate, the pulse rate, too, can be found at the rate of once every second.

The method in which the pulse interval is calculated once every second by setting the pulse interval calculation section of four seconds with a shift of one second, that is, allowing an overlap of three seconds, is described above, and similar processing can be applied to the determination section for determining a pulse wave abnormality period. In the flowchart of FIG. 19, an example where a determination section of 20 seconds is used at the time of finding the result of determination is shown and it is assumed that the result of determination is found once every 20 seconds. However, if the determination section is set with a shift of x seconds each, the result of determination can be found once every x seconds. In this example, x seconds may be one second as in the calculation of the pulse interval, or may be such lengths as two seconds, five seconds, or ten seconds.

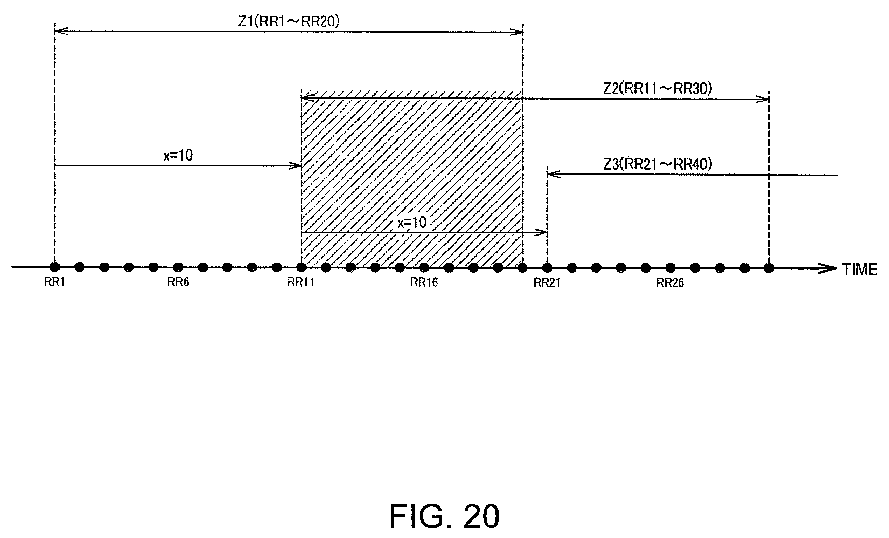

In the case where overlaps of determination sections are thus allowed, the value of one pulse interval corresponds to a plurality of determination sections. A specific example is shown in FIG. 20. In FIG. 20, it is assumed that the determination section is 20 seconds, the value x is ten seconds, and the pulse interval is calculated once every second. In this example, the value of the pulse interval at the end point of the determination section is not used in this determination section. Therefore, 20 values of the pulse interval are included in the determination section of 20 seconds. In FIG. 20, a first determination section Z1 and a second determination section Z2 next to Z1 are set. Of the pulse intervals, RR11 to RR20 are included in both Z1 and Z2. That is, a result of determination R1 on a pulse wave abnormality period found from Z1, and a result of determination R2 on a pulse wave abnormality period found from Z2 are both information based on RR11 to RR20.

In this case, as the result of determination on a pulse wave abnormality period based on RR11 to RR20, one of R1 and R2 may be used. However, since a plurality of pieces of information corresponding to RR11 to RR20 is found, the plurality of pieces of information may be combined together. As an example, the result of determination in the section corresponding to RR11 to RR20 may be decided, using the logical disjunction, logical conjunction, majority rule or the like of R1 and R2. While x=10 holds in FIG. 20, if x is smaller than that, one pulse interval value corresponds to a greater number of determination sections. For example, if x=1 holds, one pulse interval value corresponds to 20 determination sections. Therefore, the result of determination for this one pulse interval may be found using the logical disjunction, logical conduction, majority rule or the like of 20 patterns of results of determination. The length of the determination section and the length of x may be implemented with other modifications, as described above.

The feature that the determination section may be set with a shift of x seconds each similarly applies to the case of finding the reliability index (section reliability). In this case, similar settings need not be carried out for the result of determination on a pulse wave abnormality period and for the section reliability. Therefore, the length of the determination section, or x, which is the width by which the determination section is shifted at the time of setting, may be set to different values.

3.2 Specific Example of Pulse Wave Measuring Device and the Like

FIGS. 21A to 22 show an example of the appearance of the pulse wave measuring device 100 (wearable device) for gathering pulse wave information. The wearable device in the embodiment has a band 10, a case 30, and a sensor 40. The case 30 is attached to the band 10. The sensor 40 is attached to the case 30.

The band 10 is to be wound on the user's wrist so that the user can wear the wearable device. The band 10 has a band hole 12 and a buckle 14. The buckle 14 has a band insertion part 15 and a protrusion 16. The user inserts one end of the band 10 into the band insertion part 15 of the buckle 14 and inserts the protrusion 16 of the buckle 14 into the band hole 12 of the band 10, thus wearing the wearable device on the wrist. The band 10 may also be configured with a clasp instead of the buckle 14.

The case 30 is equivalent to the main body of the wearable device. Inside the case 30, various components of the wearable device such as the sensor 40 and a circuit board or the like, not shown, are provided. That is, the case 30 is a casing accommodating these components.

The case 30 is provided with a light emitting window 32. The light emitting window 32 is formed of a light-transmitting member. The case 30 provided with a light emitting device as an interface mounted on a flexible substrate. The light from the light emitting device is emitted out of the case 30 via the light emitting window 32.

The wearable device is worn by the user on the wrist, as shown in FIG. 23A, and pulse wave information (in a broad sense, biological information) is measured in the state where the wearable device is worn.

Next, an example of a specific device which implements the biological information processing system 200 according to the embodiment will be described. The biological information processing system 200 according to the embodiment may be, for example, a server system. An example of this case is shown in FIG. 23A. The biological information processing system 200 as a server system is connected to the pulse wave measuring device 100 via a network NE and acquires pulse wave information from the pulse wave measuring device 100. Since the wearable device (pulse wave measuring device 100) worn by the user needs to be small-sized and lightweight, the battery capacity, the processing capability of the processor inside the device, or the memory capacity for data is very limited. In contrast, in the server system, since resources are less limited, the processing of analyzing the pulse wave information and the processing of generating the analysis result information can be carried out at high speeds, and more data (pulse wave information or analysis result information) can be held.

It suffices that the biological information processing system 200 can acquire the pulse wave information gathered by the pulse wave measuring device 100. Therefore, the biological information processing system 200 is not limited to being connected directly to the pulse wave measuring device 100. For example, the pulse wave measuring device 100 may be connected to another processing device 400, and the biological information processing system 200 may be connected to the processing device 400 via the network NE, as shown in FIG. 23B. The processing device 400 in the case may be, for example, a mobile terminal device such as a smartphone used by a user wearing the pulse wave measuring device 100. For the connection between the pulse wave measuring device 100 and the processing device 400, an equivalent to the network NE may be used, and near field wireless communication or the like may also be used.

The biological information processing system 200 according to the embodiment may be implemented by a processing device such as a smartphone (in a narrow sense, a mobile terminal device), instead of the server system. An example of the configuration in this case is shown in FIG. 23C. The mobile terminal device such as a smartphone often has limitations to its processing capability, memory area and battery capacity, compared with the server system. However, in consideration of the recent improvement in performance, it may be possible to secure sufficient processing capability and the like. Therefore, if the requirements of processing capability and the like are met, a smartphone or the like can be used as the biological information processing system 200 according to the embodiment, as shown in FIG. 23C.

Moreover, when improvement in terminal performance or the way of using the device or the like is considered, an embodiment in which the pulse wave measuring device 100 includes the biological information processing system 200 according to the embodiment is not precluded. In this case, the acquirer 210 receives (acquires) information from the pulse wave sensor 110 in the same device. In the case where the biological information processing system 200 is installed in the pulse wave measuring device 100, this biological information processing system 200 hardly needs to perform analysis, saving or the like of data with respect to a large number of users, and may only have to handle one or a smaller number of user using the pulse wave measuring device 100. That is, it is highly possible that, even with the processing capability of the pulse wave measuring device 100, requests from users can be dealt with.

That is, the technique in the embodiment can be applied to a biological information processing device (biological information analysis device, biological information measuring device, biological information detecting device) including an acquirer 210 which acquires pulse wave information, a processor 230 which performs analysis processing on the pulse wave information to generate analysis result information, and an output 250 which outputs the generated analysis result information. The processor 230 of the biological information processing device generates information for identifiably displaying a pulse wave abnormality period detected on the basis of the analysis processing and for displaying at least one of action information psychological state information, external environment information and location information of the user, as described above.

Alternatively, the technique in the embodiment can be applied to a terminal device (biological information processing device, biological information analysis device, biological information measuring device, biological information detecting device) including a receiver which receives analysis result information about pulse wave information, a processor 230 which performs display processing on the received analysis result information, and a display on which the analysis result information is displayed. The processor 230 performs display processing in which the analysis result information is displayed on a first screen, and performs display processing in which original information used to generate the analysis result information is displayed on a second screen when the user carries out a predetermined instruction operation.

In the above description, the biological information processing system 200 is implemented by one of the server system, the processing device 400 and the pulse wave measuring device 100. However, this configuration is not limiting. For example, the acquisition of the pulse wave information, the analysis processing on the pulse wave information, the processing of generating the analysis result information, and the processing of outputting the analysis result information may be implemented by distributed processing among a plurality of devices. Specifically, the biological information processing system 200 may be implemented by at least two or more of the server system, processing device 400 and the pulse wave measuring device 100. Alternatively, like the presentation device 300 of FIG. 16, another device may perform a part of the processing by the biological information processing system 200. The biological information processing system 200 according to the embodiment can be implemented by various devices (or combinations of devices).

The biological information processing system 200, the biological information processing device and the terminal device or the like according to the embodiment include a memory which stores information (for example, programs and various data), and a processor which operates on the basis of the information stored in the memory. The processor performs acquisition processing in which pulse wave information is acquired, processing in which analysis processing on the pulse wave information is performed to generate analysis result information, and output processing in which the analysis result information that is generated is outputted. The processor generates analysis result information for identifiably displaying a pulse wave abnormality period detected on the basis of the analysis processing and for displaying at least one of action information, psychological state information, external environment information and location information of the user.

Alternatively, the processor performs acquisition processing in which pulse wave information is acquired, processing in which analysis processing on the pulse wave information is performed to generate analysis result information, and display processing on the analysis result information. The processor, in the display processing, performs display processing on a first screen where the analysis result information is displayed, and performs display processing on a second screen where original information used to generate the analysis result information is displayed when the user carries out a predetermined instruction operation.

The functions of the individual parts of the processor may be implemented, for example, by individual pieces of hardware, or the functions of the individual parts may be implemented by an integrated piece of hardware. For example, the processor may be a CPU (central processing unit). However, the processor is not limited to the CPU, and various kinds of processors such as GPU (graphics processing unit) or DSP (digital signal processor) can be used. The processor may also be a hardware circuit based on ASIC (application specific integrated circuit). The memory may be, for example, a semiconductor memory such as an SRAM or DRAM, a register, magnetic memory such as a hard disk drive, or an optical memory such as an optical disk drive. For example, the memory stores a computer-readable command, and as the processor executes the command, a function of each part of the image processing device. The command in this case may be a command of a command set forming a program, or may be a command which instructs the hardware circuit of the processor to carry out an operation.

The operations in the embodiment are implemented as follows, for example. The processor acquires pulse wave information from the pulse wave sensor 110 and stores the pulse wave information in the memory. The processor then reads out the pulse wave information from the memory, performs analysis processing on the pulse wave information to generate analysis result information, and stores the analysis result information in the memory. In the analysis processing in this case, the processor performs processing of finding a pulse interval and storing the pulse interval in the memory, or processing of finding a reliability index and storing the reliability index in the memory, or the like. Then, the processor reads out the analysis result information from the memory and performs output processing on the analysis result information or display processing on the analysis result information.

Each part of the biological information processing system 200 and the biological information processing device according to the embodiment is implemented as a module of a program which operates on the processor. For example, the acquirer 210 is implemented as an acquisition module which acquired pulse wave information. The processor 230 is implemented as a processing module which performs analysis processing on the pulse wave information to generate analysis result information. The output 250 is implemented as an output module which outputs the generated analysis result information. The display processor is implemented as a display processing module on the analysis result information.

The pulse wave measuring device worn by the user may be made up of a pulse wave sensor, and a communication device which communicates a pulse wave sensor signal from the pulse wave sensor via wired or wireless communications. In this case, the program according to the embodiment is read out and executed by the processor (for example, CPU) of a biological information processing system which provided separately from the pulse wave measuring device and which receives the pulse wave sensor signal from the communication device. This biological information processing system may be a system which is not expected to be worn by the user, such as a PC, or may be a system which is expected to be worn (carried) by the user, such as a smartphone. Also, a server system connected via a network such as the internet may be used as the biological information processing system.

In the case where the pulse wave measuring device and the biological information processing system which executes the program are separately provided, the display used to present analysis result information or the like to the user is provided an arbitrary position. For example, the information may be displayed on the display of the biological information processing system. Alternatively, a display may be provided in the pulse wave measuring device, and pulsation information outputted from the biological information processing system may be displayed on the display. Also, the information may be displayed on the display of a different device (for example, an arbitrary client device or the like in the case where a server system is used as the biological information processing system).

The technique in the embodiment can also be applied to a method for generating analysis result information (method for producing analysis result information) in which processing of acquiring pulse wave information is performed and then an analysis processing on the pulse wave information is performed, thus generating analysis result information for identifiably displaying a pulse wave abnormality period detected on the basis of the analysis processing and for displaying at least one of action information, psychological state information, external environment information and location information of the user.

By using this generation method (production method), it is possible to display a pulse wave abnormality period and thus display a period suspected of having an occurrence of arrhythmia in a visually intelligible form and to present, to the doctor, information that serves as a clue to specifying a factor in the arrhythmia in association with the pulse wave abnormality period.

While the embodiment has been described in detail above, a person skilled in the art can readily understand that various modifications can be made without substantially departing from the new matters and advantageous effects of the invention. Therefore, all such modifications are understood as included in the scope of the invention. For example, a term described at least once along with a different term having a broader meaning or the same meaning in the specification or drawings can be replaced with the different term in any part of the specification or drawings. Also, the configurations and operations of the biological information processing system and the like are not limited to those described in the embodiment and can be implemented with various modifications.

**Claims**

1. A biological information processing system comprising
   a processor including hardware, wherein
   the processor performs:

   acquisition processing in which pulse wave information is acquired;
   processing in which analysis processing on the pulse wave information is performed to generate analysis result information; and
   output processing in which the analysis result information that is generated is outputted, and

   the processor generates the analysis result information for identifiably displaying a pulse wave abnormality period detected on the basis of the analysis processing and for displaying at least one of action information, psychological state information, external environment information and location information of a user.

2. The biological information processing system according to claim 1, wherein
   the processor generates the analysis result information for displaying the action information which is information indicating whether the user is in a sleeping state or in an awake state.

3. The biological information processing system according to claim 2, wherein
   the processor generates the analysis result information for displaying the action information as information for determination on whether a sympathetic nerve-dependent arrhythmia or a parasympathetic nerve-dependent arrhythmia occurs during the pulse wave abnormality period.

4. The biological information processing system according to claim 1, wherein
   the processor generates the analysis result information for displaying the psychological state information which is stress information of the user.

5. The biological information processing system according to claim 4, wherein
   the processor generates the analysis result information for displaying the psychological state information as information for determination on whether a stress-induced arrhythmia occurs during the pulse wave abnormality period or not.

6. The biological information processing system according to claim 1, wherein
   the processor generates the analysis result information for displaying the external environment information which is at least one of temperature information, barometric pressure information, external light information, and ambient sound information.

7. The biological information processing system according to claim 6, wherein
   the processor generates the analysis result information for displaying the external environment information which is one of the temperature information for determination on a temperature-induced arrhythmia, the barometric pressure information for determination on a barometric pressure-induced arrhythmia, the external light information for determination on an external light-induced arrhythmia, and the ambient sound information for determination on an ambient sound-induced arrhythmia,
   as information for determination on what external environment induces an arrhythmia occurring during the pulse wave abnormality period.

8. The biological information processing system according to claim 6, wherein
   the temperature information is temperature difference information between body surface temperature of the user and external temperature.

9. The biological information processing system according to any one of claims 1 to 8, wherein
   the processor generates information for displaying at least one of a pulse waveform, a pulse interval waveform and pulse interval distribution information during a measurement period, as the analysis result information.

**10.** The biological information processing system according to any one of claims 1 to 9, wherein the processor generates information for displaying a reliability index of measurement of the pulse wave information during the measurement period, as the analysis result information.

**11.** The biological information processing system according to any one of claims 1 to 10, wherein the processor generates information for displaying a display object indicating the pulse wave abnormality period, as the analysis result information.

**12.** The biological information processing system according to any one of claims 1 to 11, wherein the processor generates information for displaying the pulse wave abnormality period and a period that is not the pulse wave abnormality period, in different image display forms from each other, as the analysis result information.

**13.** The biological information processing system according to any one of claims 1 to 12, wherein the processor generates second analysis result information for displaying at least one of a pulse waveform and a pulse interval waveform during a predetermined section in the pulse wave abnormality period.

**14.** A biological information processing device comprising
a processor including hardware, wherein
the processor performs:

acquisition processing in which pulse wave information is acquired;
processing in which analysis processing on the pulse wave information is performed to generate analysis result information; and
output processing in which the analysis result information that is generated is outputted, and

the processor generates the analysis result information for identifiably displaying a pulse wave abnormality period detected on the basis of the analysis processing and for displaying at least one of action information, psychological state information, external environment information and location information of a user.

**15.** A method for generating analysis result information, the method comprising:

performing processing in which pulse wave information is acquired; and
performing analysis processing on the pulse wave information and thus generating analysis result information for identifiably displaying a pulse wave abnormality period detected on the basis of the analysis processing and for displaying at least one of action information, psychological state information, external environment information and location information of a user.

BIOLOGICAL INFORMATION PROCESSING SYSTEM    200

210                         230                         250

ACQUIRER  →  PROCESSOR  →  OUTPUT (DISPLAY PROCESSOR)

PULSE WAVE INFORMATION                         VIEWING INFORMATION

# FIG. 1

FIG. 2

RUNNING —
WALKING —
REST —
SLEEP —

TIME

FIG. 3A

TIME

FIG. 3B

TIME

FIG. 3C

FIG. 4

AMOUNT OF ACTIVITY

TIME

## FIG. 5A

STRESS INFORMATION

TIME

## FIG. 5B

TEMPERATURE INFORMATION

TIME

## FIG. 5C

OTHER

WORKPLACE

HOME

TIME

## FIG. 5D

FIG. 6

#01 : xx/xx xx:xx～xx/xx yy:xx

FIG. 7

# FIG. 8A

RUNNING
WALKING
REST
SLEEP

LF/HF

HF
LF/HF

100
50
0

HF

AF RATE
%

100
50
0

0  4  8  12  16  20  24  4  8  12  16  20  24  4  8  12  16  20  24
2/8                2/9    TIME AND DATE    2/10              2/11

# FIG. 8B

PRESCRIPTION DRUG    WARFARIN    WARFARIN    WARFARIN    WARFARIN    WARFARIN    D2

TOTAL
DURATION OF    TIME                          BETA BLOCKER    BETA BLOCKER    BETA BLOCKER
OCCURRENCE

140    24                1            2            3            4            5
120    20
100    16
80    14                                                                            D1
60    12
40    8
20    4
10    0

2/4  5  6  7  6/5  6  7  9/1  2  3  4  12/3  4  5  6  2/8  9  10
DATE OF TEST

42

EP 3 081 157 A1

FIG. 9

43

# FIG. 10A

# FIG. 10B

xx/xx xx:xx:xx～xx:xx:xx

ACCELERATION

PULSE WAVE

PULSE
INTERVAL

# FIG. 11

xx/xx xx:xx

ACCELERATION

PULSE WAVE

PULSE
INTERVAL

FIG. 12

xx/xx xx:xx

ACCELERATION

PULSE WAVE

PULSE
INTERVAL

FIG. 13

BRAIN WAVE ANALYSIS REPORT

SUBJECT ID : xxxxxx
START OF TEST : YEAR xxxx  MONTH xx  DAY  XX
END OF TEST : YEAR xxxx  MONTH xx  DAY  XX
DURATION OF TEST : xx : xx

1. STATISTICAL SUMMARY

AVERAGE PULSE RATE : xxx[bpm]
MAXIMUM PULSE RATE : xxx[bpm]  @ xx/xx  xx:xx:xx
MINIMUM PULSE RATE : xxx[bpm]  @ xx/xx  xx:xx:xx
RATE OF TOTAL DURATION OF
OCCURRENCE OF ATRIAL FIBRILLATION : xx%

AF EPISODE : xx TIMES (Total : xx/Ave.  xx MINUTES)
MAXIMUM DURATION : xx MINUTES (xx/xx  xx:xx:xx~)

| | TOTAL PULSE RATE | AVERAGE PULSE RATE | TOTAL DURATION OF OCCURRENCE OF ATRIAL FIBRILLATION | RATE |
|---|---|---|---|---|
| 1日目 | xxx[bpm] | xxx[bpm] | xx:xx | xx% |
| 2日目 | xxx[bpm] | xxx[bpm] | xx:xx | xx% |
| 3日目 | xxx[bpm] | xxx[bpm] | xx:xx | xx% |
| ⋮ | ⋮ | ⋮ | ⋮ | ⋮ |

| RATIO OF OCCURRENCE OF ATRIAL FIBRILLATION | 6:00~12:00 | 12:00~18:00 | 18:00~0:00 | 0:00~6:00 |
|---|---|---|---|---|
| | xx.x% | xx.x% | xx.x% | xx.x% |

OTHER ARRHYTHMIAS :

2. TREND GRAPH

#01 : xx/xx  xx:xx～xx/xx  yy:xx

PULSE RATE

RELIABILITY
BODY MOTION
WEARING

Af

B7          B8  B9          TIME

#02 : xx/xx  yy:xx～xx/xx  zz:xx

FIG. 14

FIG. 15

FIG. 16

FIG. 17

FIG. 18A

FIG. 18B

START

ACQUIRE LOG DATA ～S101

S102
DATA FINISHED?
YES

NO

BODY MOTION NOISE
REDUCTION PROCESSING ～S103

S104
PULSE INTERVAL
CALCULATION SECTION ACCUMULATED? NO

YES

CALCULATE PULSE INTERVAL ～S105

S106
ACCUMULATED BY
AMOUNT CORRESPONDING DETERMINATION
SECTION? NO

YES

RECORD RESULT OF PULSE INTERVAL
STATISTICAL PROCESSING
(DETERMINATION ON ARRHYTHMIA) ～S107

RECORD RESULT OF DETERMINATION
ON SECTION RELIABILITY ～S108

END

FIG. 19

FIG. 20

FIG. 21A

FIG. 21B

FIG. 22

FIG. 23A          FIG. 23B          FIG. 23C

EP 3 081 157 A1

FIG. 24

Europäisches Patentamt

European Patent Office

Office européen des brevets

# EUROPEAN SEARCH REPORT

Application Number

EP 16 16 5590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/257123 A1 (WATANABE SHINICHIRO [JP]) 11 September 2014 (2014-09-11) | 1,9-15 | INV. A61B5/024 |
| Y | * paragraphs [0047], [0054] - [0071] * <br> * paragraphs [0089], [0114] - [0117] * <br> * paragraphs [0124] - [0126] * <br> * paragraphs [0132], [0135], [0140] * <br> * figures * | 2-8 | ADD. A61B5/046 A61B5/11 A61B5/16 |
| Y | EP 2 524 647 A1 (MUZET ALAIN GILLES [FR]) 21 November 2012 (2012-11-21) <br> * paragraphs [0083] - [0092] * <br> * paragraphs [0099] - [0107] * <br> * paragraphs [0124] - [0141] * <br> * paragraph [0171] * <br> * figures * | 2-8 | |
| A | US 2009/227879 A1 (MATSUMOTO CHIKAKO [JP]) 10 September 2009 (2009-09-10) <br> * paragraphs [0033], [0059] - [0064] * <br> * paragraphs [0113] - [0121] * <br> * figures * | 1-15 | |

TECHNICAL FIELDS SEARCHED (IPC)

A61B

The present search report has been drawn up for all claims

| Place of search | Date of completion of the search | Examiner |
|---|---|---|
| The Hague | 25 August 2016 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 16 5590

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2016

| Patent document cited in search report | | | Publication date | Patent family member(s) | | | Publication date |
|---|---|---|---|---|---|---|---|
| US 2014257123 | A1 | | 11-09-2014 | CN | 104027110 | A | 10-09-2014 |
| | | | | JP | 2014171660 | A | 22-09-2014 |
| | | | | US | 2014257123 | A1 | 11-09-2014 |
| EP 2524647 | A1 | | 21-11-2012 | CN | 103717125 | A | 09-04-2014 |
| | | | | EP | 2524647 | A1 | 21-11-2012 |
| | | | | EP | 2712300 | A1 | 02-04-2014 |
| | | | | JP | 2014516681 | A | 17-07-2014 |
| | | | | KR | 20140058441 | A | 14-05-2014 |
| | | | | RU | 2013156072 | A | 27-06-2015 |
| | | | | US | 2014088378 | A1 | 27-03-2014 |
| | | | | WO | 2012156427 | A1 | 22-11-2012 |
| US 2009227879 | A1 | | 10-09-2009 | JP | 5061966 | B2 | 31-10-2012 |
| | | | | JP | 2009207810 | A | 17-09-2009 |
| | | | | US | 2009227879 | A1 | 10-09-2009 |

EPO FORM P0459

For more details about this annex : see Official Journal of the European Patent Office, No. 12/82

**EP 3 081 157 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015084708 A
- JP 2015084709 A
- JP 2013055982 A